# EUROPÄISCHE PATENTSCHRIFT

(11) **EP 1 685 123 B1**
(45) Veröffentlichungstag und Bekanntmachung des Hinweises auf die Patenterteilung: **19.08.2009**
(21) Anmeldenummer: 04790948.6
(22) Anmeldetag: 28.10.2004
(51) Int. Cl.: C07D 401/04, A61K 31/517, A61P 31/12

(54) **SUBSTITUIERTE DIHYDROCHINAZOLINE II**
SUBSTITUTED DIHYDROQUINAZOLINES II
DIHYDROQUINAZOLINES II SUBSTITUEES

(30) Priorität: 11.11.2003 DE 10352499
(43) Veröffentlichungstag der Anmeldung: 02.08.2006
(73) Patentinhaber: Bayer HealthCare AG, 51368 Leverkusen (DE)
(72) Erfinder: WUNBERG, Tobias, 42699 Solingen (DE); BAUMEISTER, Judith, 2800 Mechelen (BE); JESKE, Mario, 42699 Solingen (DE); NELL, Peter, 42115 Wuppertal (DE); NIKOLIC, Susanne, 40789 Monheim (DE); SÜßMEIER, Frank, 42277 Wuppertal (DE); ZIMMERMANN, Holger, 42113 Wuppertal (DE); GROSSER, Rolf, 51373 Leverkusen (DE); HEWLETT, Guy, 42327 Wuppertal (DE); KELDENICH, Jörg, 42113 Wuppertal (DE); LANG, Dieter, 42553 Velbert (DE); HENNINGER, Kerstin, 42115 Wuppertal (DE)
(86) Internationale Anmeldenummer: PCT/EP2004/012175
(87) Internationale Veröffentlichungsnummer: WO 2005/047278

(56) Entgegenhaltungen:
- WO-A-99/41253
- WO-A-20/04041790
- WO-A-20/04072048
- TAKAO SAITO, KENSAKU TSUDA, YOJI SAITO: "A Facile and Efficient Carbodiimide-Mediated Synthesis of Dihydroquinazolines via a Tandem Nucleophilic Addition-Intramolecular Hetero Conjugate Addition Annulation Strategy" TETRAHEDRON LETTERS, Bd. 37, Nr. 2, 1996, Seiten 209-212, XP002326650
- FENGJIANG WANG, JAMES R. HAUSKE: "Solid-Phase Synthesis of 3,4-Dihydroquinazoline" TETRAHEDRON LETTERS, Bd. 38, Nr. 50, 1997, Seiten 8651-8654, XP002326651
- YONG SUP LEE, BUM HOON LEE, SEONG JUN PARK, SOON BANG KANG, HYEWHON RHIM, JIN-YONG PARK, JUNG-HA LEE, SEONG WOO JEONG, JAE YEOL LE: "3,4-Dihydroquinazoline derivatives as novel selective T-type Ca2+ channel blockers" BIOORGANIC & MEDICINAL CHEMISTRY LETTERS, Bd. 14, 2004, Seiten 3379-3384, XP002326652

## Beschreibung

Die Erfindung betrifft substituierte Dihydrochinazoline und Verfahren zu ihrer Herstellung, ihre Verwendung zur Behandlung und/oder Prophylaxe von Krankheiten sowie ihre Verwendung zur Herstellung von Arzneimitteln zur Behandlung und/oder Prophylaxe von Krankheiten, insbesondere zur Verwendung als antivirale Mittel, insbesondere gegen Cytomegaloviren.

Die WO99/41253 beschreibt antivirale Verbindungen auf Basis von Pyrimidinderivaten. Die Synthese von Dihydrochinazolinen ist beschrieben in Saito T., et al. Tetrahedron Lett., 1996, 37, 209-212, und in Wang F., et al. Tetrahedron Lett., 1997, 38, 8651-8654. Desweiteren wurden Dihydrochinazoline von Y.S. Lee et al. Bioorg. Med. Chem. Lett. 2004, 14, 3379-3384 als Kalziumkanalinhibitoren beschrieben.

Auf dem Markt sind zwar strukturell andersartige antiviral wirkende Mittel vorhanden, aber die gegenwärtig verfügbaren Therapien mit Ganciclovir, Valganciclovir, Foscamet und Cidofovir sind mit schweren Nebenwirkungen verbunden, z. B. Nephrotoxizität, Neutropenie oder Thrombozytopenie. Zudem kann es regelmäßig zu einer Resistenzentwicklung kommen. Neue Mittel für eine wirksame Therapie sind daher wünschenswert.

Eine Aufgabe der vorliegenden Erfindung ist es daher, neue Verbindungen mit gleicher oder verbesserter antiviraler Wirkung zur Behandlung von viralen Infektionskrankheiten bei Menschen und Tieren zur Verfügung zu stellen.

Überraschenderweise wurde gefunden, dass die in der vorliegenden Erfindung beschriebenen substituierten Dihydrochinazoline antiviral hochwirksam sind.

Gegenstand der Erfindung sind Verbindungen der Formel 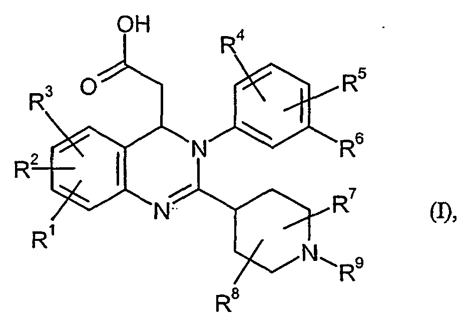 in welcher
- R¹, R² und R³: unabhängig voneinander für Wasserstoff, Alkyl, Alkoxy, Carboxyl, Alkylcarbonyl, Alkoxycarbonyl, Aminocarbonyl, Trifluormethyl, Halogen; Cyano, Hydroxy oder Nitro stehen,
- R⁴ und R⁵: unabhängig voneinander für Wasserstoff, Alkyl, Alkoxy, Alkylthio, Cyano, Halogen, Nitro, Trifluormethyl oder Trifluormethoxy stehen,
- R⁶: für Alkyl, Cyano, Halogen, Nitro oder Trifluormethyl steht,
- R⁷ und R⁸: unabhängig voneinander für Wasserstoff, Halogen, Alkyl oder Alkoxy stehen und
- R⁹: für Aryl oder 1,3-Benzodioxol-5-yl steht, worin Aryl und 1,3-Benzodioxol-5-yl substituiert sein können mit 1 bis 3 Substituenten, wobei die Substituenten unabhängig voneinander ausgewählt werden aus der Gruppe, bestehend aus Alkoxy, Alkylthio, Carboxyl, Alkylcarbonyl, Alkoxycarbonyl, Aminocarbonyl, Trifluormethyl, Halogen, Carbamoyl, Cyano, Hydroxy, Amino, Alkylamino, Nitro und gegebenenfalls Hydroxy-substituiertes Alkyl,
und ihre Salze, ihre Solvate und die Solvate ihrer Salze.

Erfmdungsgemäße Verbindungen sind die Verbindungen der Formel (I) und deren Salze, Solvate und Solvate der Salze, nachfolgend als Ausführungsbeispiel(e) genannten Verbindungen und deren Salze, Solvate und Solvate der Salze, soweit es sich bei den von Formel (I) umfassten, nachfolgend genannten Verbindungen nicht bereits um Salze, Solvate und Solvate der Salze handelt.

Die erfindungsgemäßen Verbindungen können in Abhängigkeit von ihrer Struktur in stereoisomeren Formen (Enantiomere, Diastereomere) existieren. Die Erfindung betrifft deshalb die Enantiomeren oder Diastereomeren und ihre jeweiligen Mischungen. Aus solchen Mischungen von Enantiomeren und/oder Diastereomeren lassen sich die stereoisomer einheitlichen Bestandteile in bekannter Weise isolieren.

Sofern die erfindungsgemäßen Verbindungen in tautomeren Formen vorkommen können, umfasst die vorliegende Erfindung sämtliche tautomere Formen.

Als Salze sind im Rahmen der vorliegenden Erfindung physiologisch unbedenkliche Salze der erfindungsgemäßen Verbindungen bevorzugt. Umfasst sind aber auch Salze, die für pharmazeutische Anwendungen selbst nicht geeignet sind aber beispielsweise für die Isolierung oder Reinigung der erfindungsgemäßen Verbindungen verwendet werden können.

Physiologisch unbedenkliche Salze der erfindungsgemäßen Verbindungen umfassen Säureadditionssalze von Mineralsäuren, Carbonsäuren und Sulfonsäuren, z.B. Salze der Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Trifluoressigsäure, Propionsäure, Milchsäure, Weinsäure, Äpfelsäure, Zitronensäure, Fumarsäure, Maleinsäure und Benzoesäure.

Physiologisch unbedenkliche Salze der erfindungsgemäßen Verbindungen umfassen auch Salze üblicher Basen, wie beispielhaft und vorzugsweise Alkalimetallsalze (z.B. Natrium- und Kaliumsalze), Erdalkalisalze (z.B. Calcium- und Magnesiumsalze) und Ammoniumsalze, abgeleitet von Ammoniak oder organischen Aminen mit 1 bis 16 C-Atomen, wie beispielhaft und vorzugsweise Ethylamin, Diethylamin, Triethylamin, Ethyldiisopropylamin, Monoethanolamin, Diethanolamin, Triethanolamin, Dicyclohexylamin, Dimethylaminoethanol, Prokain, Dibenzylamin, N-Methylmorpholin, Arginin, Lysin, Ethylendiamin und N-Methylpiperidin.

Als Solvate werden im Rahmen der Erfindung solche Formen der erfindungsgemäßen Verbindungen bezeichnet, welche in festem oder flüssigem Zustand durch Koordination mit Lösungsmittelmolekülen einen Komplex bilden. Hydrate sind eine spezielle Form der Solvate, bei denen die Koordination mit Wasser erfolgt.

Im Rahmen der vorliegenden Erfindung haben die Substituenten, soweit nicht anders spezifiziert, die folgende Bedeutung:

Alkyl per se und "Alk" und "Alkyl" in Alkoxy, Alkylthio, Alkylamino, Alkylcarbonyl und Alkoxycarbonyl stehen für einen linearen oder verzweigten Alkylrest mit in der Regel 1 bis 6 ("C₁-C₆-Alkyl"), vorzugsweise 1 bis 4, besonders bevorzugt 1 bis 3 Kohlenstoffatomen, beispielhaft und vorzugsweise für Methyl, Ethyl, n-Propyl, Isopropyl, tert.-Butyl, n-Pentyl und n-Hexyl.

Alkoxy steht beispielhaft und vorzugsweise für Methoxy, Ethoxy, n-Propoxy, Isopropoxy, tert.-Butoxy, n-Pentoxy und n-Hexoxy.

Alkylthio steht beispielhaft und vorzugsweise für Methylthio, Ethylthio, n-Propylthio, Isopropylthio, tert.-Butylthio, n-Pentylthio und n-Hexylthio.

Alkylcarbonyl steht beispielhaft und vorzugsweise für Acetyl und Propanoyl.

Alkylamino steht für einen Alkylaminorest mit einem oder zwei (unabhängig voneinander gewählten) Alkylsubstituenten, beispielhaft und vorzugsweise für Methylamino, Ethylamino, n-Propylamino, Isopropylamino, tert.-Butylamino, n-Pentylamino, n-Hexylamino, N,N-Dimethylamino, N,N-Diethylamino, N-Ethyl-N-methylamino, N-Methyl-N-n-propylamino, N-Isopropyl-N-n-propylamino, N-t-Butyl-N-methylamino, N-Ethyl-N-n-pentylamino und N-n-Hexyl-N-methylamino. C₁-C₃-Alkylamino steht beispielsweise für einen Monoalkylaminorest mit 1 bis 3 Kohlenstoffatomen oder für einen Dialkylaminorest mit jeweils 1 bis 3 Kohlenstoffatomen pro Alkylsubstituent.

Alkoxycarbonyl steht beispielhaft und vorzugsweise für Methoxycarbonyl, Ethoxycarbonyl, n-Propoxycarbonyl, Isopropoxycarbonyl, tert.-Butoxycarbonyl, n-Pentoxycarbonyl und n-Hexoxycarbonyl.

Aryl steht für einen mono- bis tricyclischen aromatischen, carbocyclischen Rest mit in der Regel 6 bis 14 Kohlenstoffatomen; beispielhaft und vorzugsweise für Phenyl, Naphthyl und Phenanthrenyl.

Halogen steht für Fluor, Chlor, Brom und Jod.

Ein Symbol * an einem Kohlenstoffatom bedeutet, dass die Verbindung hinsichtlich der Konfiguration an diesem Kohlenstoffatom in enantiomerenreiner Form vorliegt, worunter im Rahmen der vorliegenden Erfmdung eine Enantiomerenüberschuss (enantiomeric excess) von mehr als 90 % verstanden wird (> 90%ee).

Bevorzugt im Rahmen der vorliegenden Erfindung sind Verbindungen der Formel (I),
in welcher
- R¹, R² und R³: unabhängig voneinander für Wasserstoff, Fluor, Chlor, Cyano, Hydroxy oder Aminocarbonyl stehen,
- R⁴ und R⁵: unabhängig voneinander für Wasserstoff, Fluor, Alkyl oder Alkoxy stehen,
- R⁶: für Chlor, Nitro, Trifluormethyl, Methyl, iso-Propyl oder tert.-Butyl steht,
- R⁷ und R⁸: unabhängig voneinander für Wasserstoff oder C₁-C₃-Alkyl stehen und
- R⁹: für Phenyl oder 1,3-Benzodioxol-5-yl steht, worin Phenyl substituiert sein kann mit 1 bis 3 Substituenten, wobei die Substituenten unabhängig voneinander ausgewählt werden aus der Gruppe, bestehend aus C₁-C₆-Alkyl, C₁-C₆-Alkoxy, Carboxyl, C₁-C₆-Alkylcarbonyl, C₁-C₆-Alkoxycarbonyl, Trifluormethyl, Fluor, Chlor, Brom, Cyano, Hydroxy, Amino, C₁-C₆-Alkylamino und Nitro,
und ihre Salze, ihre Solvate und die Solvate ihrer Salze.

Bevorzugt im Rahmen der vorliegenden Erfindung sind Verbindungen der Formel (I),
in welcher
- R¹, R² und R³: unabhängig voneinander für Wasserstoff, Methyl, Fluor, Chlor, Cyano, Hydroxy oder Aminocarbonyl stehen,
- R⁴ und R⁵: unabhängig voneinander für Wasserstoff, Fluor, C₁-C₄-Alkyl oder C₁-C₄-Alkoxy stehen,
- R⁶: für Chlor, Nitro, Trifluormethyl, Methyl, iso-Propyl oder tert.-Butyl steht,
- R⁷ und R⁸: unabhängig voneinander für Wasserstoff oder C₁-C₃-Alkyl stehen und
- R⁹: für Phenyl oder 1,3-Benzodioxol-5-yl steht, worin Phenyl substituiert sein kann mit 1 bis 3 Substituenten, wobei die Substituenten unabhängig voneinander ausgewählt werden aus der Gruppe, bestehend aus C₁-C₆-Alkyl, C₁-C₆-Alkoxy, Carboxyl, C₁-C₆-Alkylcarbonyl, C₁-C₆-Alkoxycarbonyl, Trifluormethyl, Fluor, Chlor, Brom, Cyano, Hydroxy, Amino, C₁-C₆-Alkylamino und Nitro,
und ihre Salze, ihre Solvate und die Solvate ihrer Salze.

Bevorzugt im Rahmen der vorliegenden Erfindung sind auch Verbindungen der Formel (I),
in welcher
- R¹ und R²: für Wasserstoff stehen,
- R³: für Fluor steht,
- R⁴ und R⁵: unabhängig voneinander für Wasserstoff, Fluor oder Alkoxy stehen,
- R⁶: für Trifluormethyl steht,
- R⁷ und R⁸: für Wasserstoff stehen und
- R⁹: für Phenyl steht, worin Phenyl substituiert sein kann mit 1 oder 2 Substituenten, wobei die Substituenten unabhängig voneinander ausgewählt werden aus der Gruppe, bestehend aus Methyl, Methoxy, Fluor und Chlor,
und ihre Salze, ihre Solvate und die Solvate ihrer Salze.

Bevorzugt im Rahmen der vorliegenden Erfindung sind auch Verbindungen der Formel (I),
in welcher
- R¹ und R²: für Wasserstoff stehen,
- R³: für Fluor steht,
- R⁴ und R⁵: unabhängig voneinander für Wasserstoff, Fluor oder Methoxy stehen,
- R⁶: für Trifluormethyl steht,
- R⁷ und R⁸: für Wasserstoff stehen und
- R⁹: für Phenyl steht, worin Phenyl substituiert sein kann mit 1 oder 2 Substituenten, wobei die Substituenten unabhängig voneinander ausgewählt werden aus der Gruppe, bestehend aus Methyl, Methoxy, Ethoxy, Fluor und Chlor,
und ihre Salze, ihre Solvate und die Solvate ihrer Salze.

Bevorzugt im Rahmen der vorliegenden Erfindung sind auch Verbindungen der Formel (I), in welcher R¹ und R² für Wasserstoff stehen.

Bevorzugt im Rahmen der vorliegenden Erfindung sind auch Verbindungen der Formel (I), in welcher R³ an das Kohlenstoffatom in Position 6 oder in Position 8 des Chinazolin-Gerüsts gebunden ist.

Bevorzugt im Rahmen der vorliegenden Erfindung sind auch Verbindungen der Formel (I), in welcher R³ an das Kohlenstoffatom in Position 8 des Chinazolin-Gerüsts gebunden ist.

Bevorzugt im Rahmen der vorliegenden Erfindung sind auch Verbindungen der Formel (I), in welcher R³ für Fluor steht, insbesondere für ein an das Kohlenstoffatom in Position 8 des Chinazolin-Gerüstes gebundenes Fluor.

Bevorzugt im Rahmen der vorliegenden Erfindung sind auch Verbindungen der Formel (I), in welcher R⁴ und R⁵ für Wasserstoff stehen.

Bevorzugt im Rahmen der vorliegenden Erfindung sind auch Verbindungen der Formel (I), in welcher R⁴ für Wasserstoff und R⁵ für Fluor oder Alkoxy stehen.

Bevorzugt im Rahmen der vorliegenden Erfindung sind auch Verbindungen der Formel (I), in welcher R⁴ für Wasserstoff und R⁵ für Methoxy stehen.

Bevorzugt im Rahmen der vorliegenden Erfindung sind auch Verbindungen der Formel (I), in welcher R⁶ für Trifluormethyl steht.

Bevorzugt im Rahmen der vorliegenden Erfmdung sind auch Verbindungen der Formel (I), in welcher R⁶ für Methyl, iso-Propyl oder tert.-Butyl steht.

Bevorzugt im Rahmen der vorliegenden Erfindung sind auch Verbindungen der Formel (I), in welcher R⁶ für iso-Propyl oder tert.-Butyl steht.

Bevorzugt im Rahmen der vorliegenden Erfindung sind auch Verbindungen der Formel (I), in welcher R⁷ und R⁸ für Wasserstoff stehen.

Bevorzugt im Rahmen der vorliegenden Erfindung sind auch Verbindungen der Formel (I), in welcher R⁹ für Phenyl steht, worin Phenyl substituiert sein kann mit 1 bis 2 Substituenten, wobei die Substituenten unabhängig voneinander ausgewählt werden aus der Gruppe bestehend aus Methyl, Methoxy, Fluor und Chlor.

Bevorzugt im Rahmen der vorliegenden Erfmdung sind auch Verbindungen der Formel (I), in welcher R⁹ für Phenyl steht, worin Phenyl substituiert ist mit 1 bis 2 Substituenten, wobei die Substituenten unabhängig voneinander ausgewählt werden aus der Gruppe bestehend aus Methyl, Methoxy, Fluor und Chlor.

Bevorzugt im Rahmen der vorliegenden Erfindung sind auch Verbindungen der Formel (I), in welcher R⁹ für Phenyl steht, worin Phenyl in para-Position zur Verknüpfungsstelle mit dem Piperidin-Ring mit Fluor substituiert ist.

Bevorzugt im Rahmen der vorliegenden Erfindung sind auch Verbindungen der Formel (I), in welcher R⁹ für Phenyl steht, worin Phenyl in meta-Position zur Verknüpfungsstelle mit dem Piperidin-Ring mit Chlor, Methyl oder Methoxy substituiert ist.

Bevorzugt im Rahmen der vorliegenden Erfindung sind auch Verbindungen der Formel (I), in welcher R⁹ für Phenyl steht, worin Phenyl in meta-Position zur Verknüpfungsstelle mit dem Piperidin-Ring mit Methyl und in para-Position zur Verknüpfungsstelle mit dem Piperidin-Ring mit Fluor substituiert ist.

Gegenstand der Erfindung ist weiterhin ein Verfahren zur Herstellung der Verbindungen der Formel (I), wobei Verbindungen der Formel 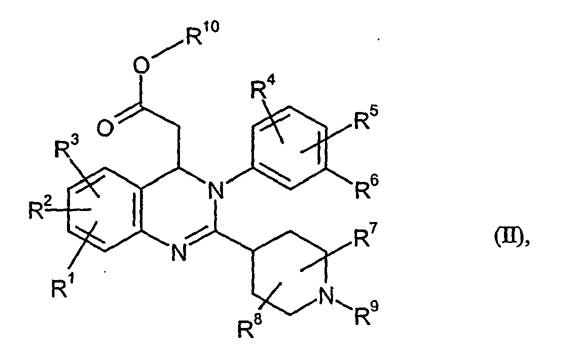 in welcher
R¹, R², R³, R⁴, R⁵, R⁶, R⁷, R⁸ und R⁹ die oben angegebene Bedeutung haben, und
R¹⁰ für Alkyl, bevorzugt Methyl oder Ethyl, steht,
mit Basen umgesetzt werden.

Die Umsetzung erfolgt im allgemeinen in inerten Lösungsmitteln, bevorzugt in einem Temperaturbereich von Raumtemperatur bis zum Rückfluss der Lösungsmittel bei Normaldruck.

Basen sind beispielsweise Alkalihydroxide wie Natrium-, Lithium- oder Kaliumhydroxid, oder Alkalicarbonate wie Cäsiumcarbonat, Natrium- oder Kaliumcarbonat, gegebenenfalls in wässriger Lösung, bevorzugt ist Natriumhydroxid in Wasser.

Inerte Lösungsmittel sind beispielsweise Halogenkohlenwasserstoffe wie Ether wie 1,2-Dimethoxyethan, Dioxan, Tetrahydrofuran, Glykoldimethylether oder Diethylenglykoldimethylether, Alkohole wie Methanol, Ethanol, n-Propanol, iso-Propanol, n-Butanol oder tert.-Butanol, oder Gemischen von Lösungsmitteln, bevorzugt ist Dioxan oder Tetrahydrofuran.

Die Verbindungen der Formel (II) sind bekannt oder können hergestellt werden, indem Verbindungen der Formel 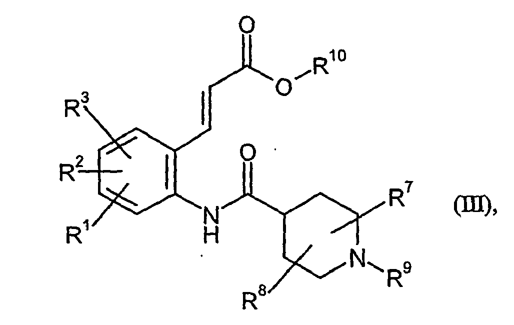 in welcher
R¹, R², R³, R⁷, R⁸, R⁹ und R¹⁰ die oben angegebene Bedeutung haben,
mit Verbindungen der Formel 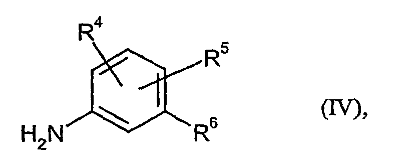 in welcher
R⁴, R⁵ und R⁶ die oben angegebene Bedeutung haben,
in Gegenwart von Phosphoroxychlorid umgesetzt werden.

Die Umsetzung erfolgt im Allgemeinen in inerten Lösungsmitteln, bevorzugt in einem Temperaturbereich von 50°C bis zum Rückfluss der Lösungsmittel bei Normaldruck.

Inerte Lösungsmittel sind beispielsweise Kohlenwasserstoffe wie Benzol, Xylol, Toluol, Hexan, Cyclohexan oder Erdölfraktionen, bevorzugt ist Toluol.

Alternativ können die Verbindungen der Formel (II) in einem zweistufigen Syntheseverfahren hergestellt werden. In der ersten Stufe werden die Verbindungen der Formel (III) mit Phosphoroxychlorid in einem inerten Lösungsmittel, bevorzugt ist Toluol, unter Rückfluss bei Normaldruck erhitzt. Das Lösungsmittel wird entfernt. In der zweiten Stufe werden die so erhaltenen Verbindungen mit Verbindungen der Formel (IV) in einem inerten Lösungsmittel, bevorzugt ist Toluol, ebenfalls unter Rückfluss bei Normaldruck umgesetzt.

Die Verbindungen der Formel (IV) sind bekannt oder lassen sich nach bekannten Verfahren aus den entsprechenden Edukten synthetisieren.

Die Verbindungen der Formel (III) sind bekannt oder können hergestellt werden, indem Verbindungen der Formel 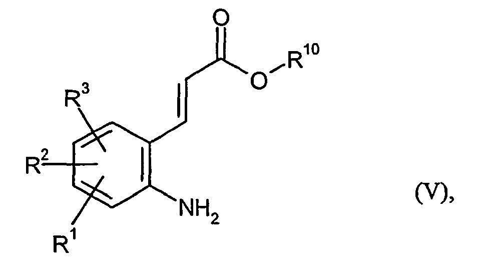 in welcher
R¹, R², R³ und R¹⁰ die oben angegebene Bedeutung haben,
mit Verbindungen der Formel 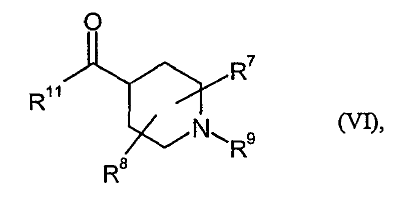 in welcher
R⁷, R⁸ und R⁹ die oben angegebene Bedeutung haben und
R¹¹ für Halogen, bevorzugt Chlor, Brom oder Iod, oder Hydroxy steht,
umgesetzt werden.

Im Falle, dass R¹¹ für Hydroxy steht,
erfolgt die Umsetzung im allgemeinen in inerten Lösungsmitteln, in Gegenwart von üblichen Kondensationsmitteln, gegebenenfalls in Gegenwart einer Base, bevorzugt in einem Temperaturbereich von Raumtemperatur bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, 1,2-Dimethoxyethan, Glykoldimethylether oder Diethylenglykoldimethylether, Kohlenwasserstoffe wie Benzol, Xylol, Toluol, Hexan, Cyclohexan oder Erdölfraktionen, oder Carbonsäureamide wie Dimethylformamid oder Dimethylacetamid, Alkylnitrile wie Acetonitril, oder Heteroaromaten wie Pyridin, oder Ethylacetat, bevorzugt sind Tetrahydrofuran, 1,2-Dichlorethan oder Methylenchlorid.

Übliche Kondensationsmittel sind beispielsweise Carbodiimide wie z.B. N,N'-Diethyl-, N,N,'-Dipropyl-, N,N'-Diisopropyl-, N,N'-Dicyclohexylcarbodiimid, N-(3-Dimethylaminoisopropyl)-N'-ethylcarbodiimid-Hydrochlorid (EDC), N-Cyclohexylcarbodiimid-N'-propyloxymethyl-Polystyrol (PS-Carbodiimid) oder Carbonylverbindungen wie Carbonyldiimidazol, oder 1,2-Oxazoliumverbindungen wie 2-Ethyl-5-phenyl-1,2-oxazolium-3-sulfat oder 2-tert.-Butyl-5-methyl-iso-xazolium-perchlorat, oder Acylaminoverbindungen wie 2-Ethoxy-1-ethoxycarbonyl-1,2-di-hydrochinolin, oder Propanphosphonsäureanhydrid, oder Isobutylchloroformat, oder Bis-(2-oxo-3-oxazolidinyl)-phosphorylchlorid oder Benzotriazolyloxy-tri(dimethylamino)phosphonium-hexafluorophosphat, oder O-(Benzotriazol-1-yl)-N,N,N',N'-tetra-methyluronium-hexafluorophosphat (HBTU), 2-(2-Oxo-1-(2H)-pyridyl)-1,1,3,3-tetrarnethyluroniumtetrafluoro-borat (TPTU) oder O-(7-Azabenzotriazol-1-yl)-N,N,N',N'-tetramethyl-uroniumhexafluorophosphat (HATU), oder 1-Hydroxybenztriazol (HOBt), oder Benzotriazol-1-yloxytris(dimethylamino)-phosphonium-hexafluoro-phosphat (BOP), oder Mischungen aus diesen.

Basen sind beispielsweise Alkalicarbonate, wie z.B. Natrium- oder Kaliumcarbonat, oder -hydrogencarbonat, oder organische Basen wie Trialkylamine z.B. Triethylamin, N-Methylmorpholin, N-Methylpiperidin, 4- Dimethylaminopyridin oder Diisopropylethylamin.

Besonders bevorzugt ist die Kombination von N-(3-Dimethylaminoisopropyl)-N'-ethyl-carbodiimid-Hydrochlorid (EDC), 1-Hydroxybenztriazol (HOBt) und Triethylamin in Dimethylformamid oder Carbonyldiimidazol in 1,2-Dichlorethan.

Im Falle, dass R¹¹ für Halogen steht,
erfolgt die Umsetzung im allgemeinen in inerten Lösungsmitteln, gegebenenfalls in Gegenwart einer Base, bevorzugt in einem Temperaturbereich von 0°C bis 50°C bei Normaldruck.

Inerte Lösungsmittel sind beispielsweise Halogenkohlenwasserstoffe wie Methylenchlorid, Trichlormethan, Tetrachlormethan, Trichlorethan, Tetrachlorethan, 1,2-Dichlorethan oder Trichlorethylen, Ether wie Diethylether, Methyl-tert.-butylether, Dioxan, Tetrahydrofuran, 1,2-Dimethoxyethan, Glykoldimethylether oder Diethylenglykoldimethylether, Kohlenwasserstoffe wie Benzol, Xylol, Toluol, Hexan, Cyclohexan oder Erdölfraktionen, oder Carbonsäureamide wie Dimethylformamid oder Dimethylacetamid, Alkylnitrile wie Acetonitril, oder Heteroaromaten wie Pyridin, oder Ethylacetat, bevorzugt sind Tetrahydrofuran, Dioxan oder Methylenchlorid.

Basen sind beispielsweise Alkalicarbonate wie Cäsiumcarbonat, Natrium- oder Kaliumcarbonat, Alkaliacetate wie Natriumacetat oder andere Basen Wie Triethylamin oder Diisopropylethylamin, bevorzugt Diisopropylethylamin oder Triethylamin.

Die Verbindungen der Formel (VI) sind bekannt oder lassen sich nach bekannten Verfahren aus den entsprechenden Edukten synthetisieren.

Die Verbindungen der Formel (V) sind bekannt oder lassen sich nach bekannten Verfahren aus den entsprechenden Edukten synthetisieren, beispielsweise durch eine Heck-Reaktion oder eine Wittig-Horner-Reaktion nach folgenden Syntheseschemata:

### Heck-Reaktion:

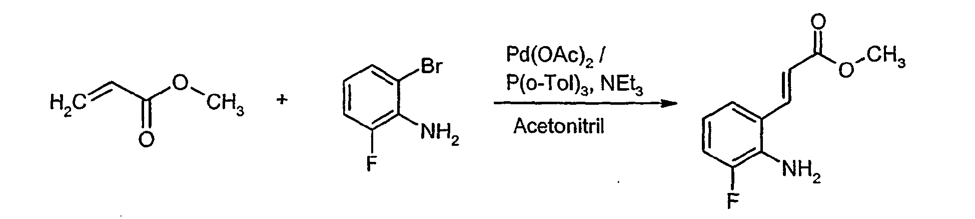

### Wittig-Horner-Reaktion:

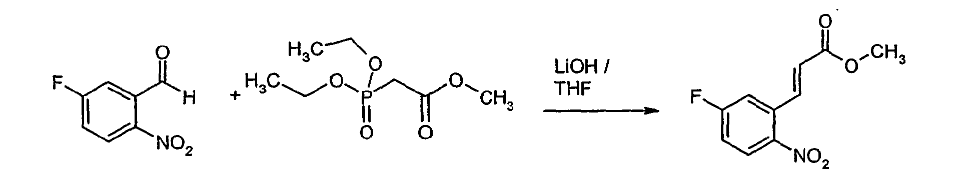

Die dafür benötigten Edukte sind bekannt oder lassen sich nach bekannten Verfahren aus den entsprechenden Edukten synthetisieren.

Die Herstellung der erfmdungsgemäßen Verbindungen kann durch das folgende Syntheseschema verdeutlicht werden.

### Syntheseschema:

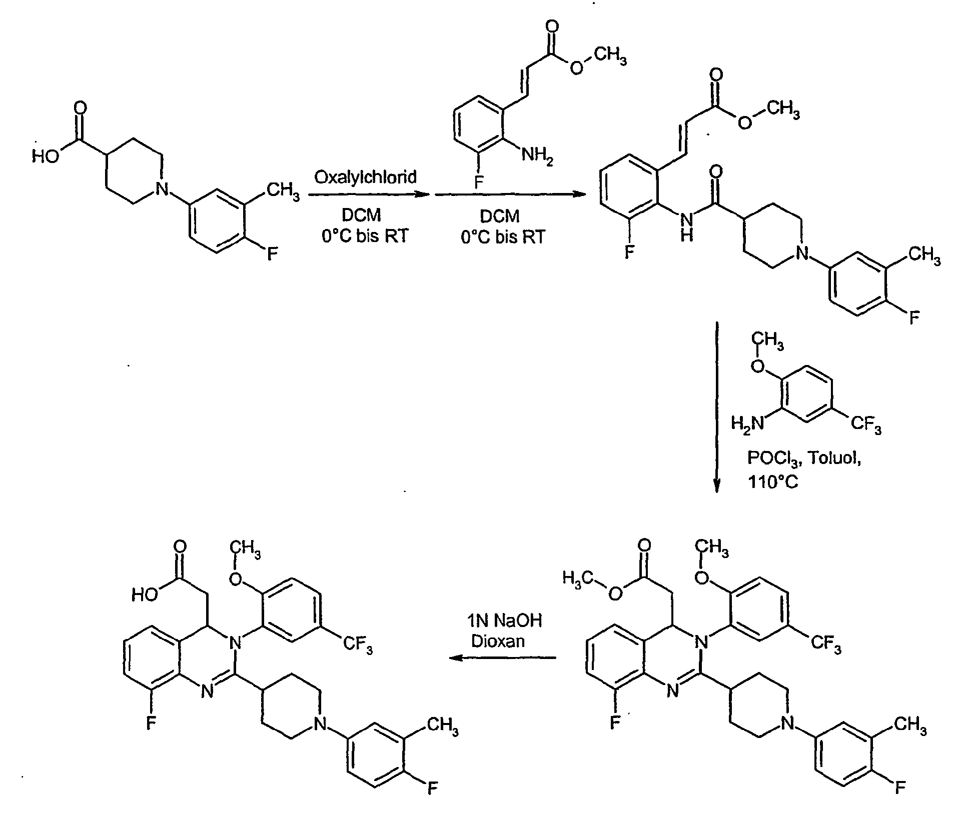

Die erfindungsgemäßen Verbindungen der allgemeinen Formel (I) zeigen ein nicht vorhersehbares, überraschendes Wirkspektrum. Sie zeigen eine antivirale Wirkung gegenüber Vertretern der Gruppe der Herpes viridae (Herpesviren), vor allem gegenüber Cytomegaloviren (CMV), insbesondere gegenüber dem humanen Cytomegalovirus (HCMV).
Als Indikationsgebiete können beispielsweise genannt werden:
1) Behandlung und Prophylaxe von HCMV-Infektionen bei AIDS-Patienten (Retinitis, Pneumonitis, gastrointestinale Infektionen).
2) Behandlung und Prophylaxe von Cytomegalovirus-Infektionen bei Knochenmark- und Organtransplantationspatienten, die an einer HCMV-Pneumonitis, -Enzephalitis, sowie an gastrointestinalen und systemischen HCMV-Infektionen oft lebensbedrohlich erkranken.
3) Behandlung und Prophylaxe von HCMV-Infektionen bei Neugeborenen und Kleinkindern.
4) Behandlung einer akuten HCMV-Infektion bei Schwangeren.
5) Behandlung der HCMV-Infektion bei immunsupprimierten Patienten bei Krebs und KrebsTherapie.
6) Behandlung von HCMV-positiven Krebspatienten mit dem Ziel, HCMV-vermittelte Tumorprogression zu verringern (vgl. J. Cinatl , et al., FEMS Microbiology Reviews 2004, 28, 59-77).

Weiterer Gegenstand der vorliegenden Erfindung ist der Einsatz der erfindungsgemäßen Verbindungen zur Behandlung und/oder Prophylaxe von Krankheiten, vor allem von Infektionen mit Viren, insbesondere den vorstehend genannten Viren, und den dadurch hervorgerufenen Infektionskrankheiten. Unter einer Virusinfektion wird nachfolgend sowohl eine Infektion mit einem Virus als auch eine durch eine Infektion mit einem Virus hervorgerufene Krankheit verstanden.

Weiterer Gegenstand der vorliegenden Erfindung ist die Verwendung der erfindungsgemäßen Verbindungen zur Behandlung und/oder Prophylaxe von Erkrankungen, insbesondere der zuvor genannten Erkrankungen.

Weiterer Gegenstand der vorliegenden Erfindung ist die Verwendung der erfindungsgemäßen Verbindungen zur Herstellung eines Arzneimittels zur Behandlung und/oder Prophylaxe von Erkrankungen, insbesondere der zuvor genannten Erkrankungen.

Bevorzugt werden die erfindungsgemäßen Verbindungen zur Herstellung von Arzneimitteln verwendet, die zur Prophylaxe und/oder Behandlung von Infektionen mit einem Vertreter der Gruppe der Herpes viridae, besonders einem Cytomegalovirus, insbesondere dem humanen Cytomegalovirus, geeignet sind.

Weiterer Gegenstand der vorliegenden Erfindung sind Arzneimittel, enthaltend mindestens eine erfindungsgemäße Verbindung und mindestens einen oder mehrere weitere Wirkstoffe, insbesondere zur Behandlung und/oder Prophylaxe der zuvor genannten Erkrankungen. Als geeigente Kombinationswirkstoffe seien beispielhaft und vorzugsweise genannt: antivirale Wirkstoffe wie Gancyclovir oder Acyclovir.

Die erfindungsgemäßen Verbindungen können systemisch und/oder lokal wirken. Zu diesem Zweck können sie auf geeignete Weise appliziert werden, wie z.B. oral, parenteral, pulmonal, nasal, sublingual, lingual, buccal, rectal, dermal, transdermal, conjunctival, otisch oder als Implantat bzw. Stent.

Für diese Applikationswege können die erfindungsgemäßen Verbindungen in geeigneten Applikationsformen verabreicht werden.

Für die orale Applikation eignen sich nach dem Stand der Technik funktionierende schnell und/oder modifiziert die erfindungsgemäßen Verbindungen abgebende Applikationsformen, die die erfindungsgemäßen Verbindungen in kristalliner und/ oder amorphisierter und/oder gelöster Form enthalten, wie z.B. Tabletten (nichtüberzogene oder überzogene Tabletten, beispielsweise mit magensaftresistenten oder sich verzögert auflösenden oder unlöslichen Überzügen, die die Freisetzung der erfindungsgemäßen Verbindung kontrollieren), in der Mundhöhle schnell zerfallende Tabletten oder Filme/Oblaten, Filme/Lyophylisate, Kapseln (beispielsweise Hart- oder Weichgelatinekapseln), Dragees, Granulate, Pellets, Pulver, Emulsionen, Suspensionen, Aerosole oder Lösungen.

Die parenterale Applikation kann unter Umgehung eines Resorptionsschrittes geschehen (z.B. intravenös, intraarteriell, intrakardial, intraspinal oder intralumbal) oder unter Einschaltung einer Resorption (z.B. intramuskulär, subcutan, intracutan, percutan oder intraperitoneal). Für die parenterale Applikation eignen sich als Applikationsformen u.a. Injektions- und Infusionszubereitungen in Form von Lösungen, Suspensionen, Emulsionen, Lyophilisaten oder sterilen Pulvern.

Für die sonstigen Applikationswege eignen sich z.B. Inhalationsarzneiformen (u.a. Pulverinhalatoren, Nebulizer), Nasentropfen, -lösungen, -sprays; lingual, sublingual oder buccal zu applizierende Tabletten, Filme/Oblaten oder Kapseln, Suppositorien, Ohren- oder Augenpräparationen, Vaginalkapseln, wäßrige Suspensionen (Lotionen, Schüttelmixturen), lipophile Suspensionen, Salben, Cremes, transdermale therapeutische Systeme, Milch, Pasten, Schäume, Streupuder, Implantate oder Stents.

Die erfindungsgemäßen Verbindungen können in die angeführten Applikationsformen überführt werden. Dies kann in an sich bekannter Weise durch Mischen mit inerten, nichttoxischen, pharmazeutisch geeigneten Hilfsstoffen geschehen. Zu diesen Hilfsstoffen zählen u.a. Trägerstoffe (beispielsweise mikrokristalline Cellulose, Laktose, Mannitol), Lösungsmittel (z.B. flüssige Polyethylenglycole), Emulgatoren und Dispergier- oder Netzmittel (beispielsweise Natriumdodecylsulfat, Polyoxysorbitanoleat), Bindemittel (beispielsweise Polyvinylpyrrolidon), synthetische und natürliche Polymere (beispielsweise Albumin), Stabilisatoren (z.B. Antioxidantien wie beispielsweise Ascorbinsäure), Farbstoffe (z.B. anorganische Pigmente wie beispielsweise Eisenoxide) und Geschmackes- und / oder Geruchskorrigentien.

Weiterer Gegenstand der vorliegenden Erfindung sind Arzneimittel, die mindestens eine erfindungsgemäße Verbindung, üblicherweise zusammen mit einem oder mehreren inerten, nichttoxischen, pharmazeutisch geeigneten Hilfsstoffen enthalten, sowie deren Verwendung zu den zuvor genannten Zwecken.

Im Allgemeinen hat es sich als vorteilhaft erwiesen, bei intravenöser Applikation Mengen von etwa 0.001 bis 10 mg/kg, vorzugsweise etwa 0.01 bis 5 mg/kg Körpergewicht zur Erzielung wirksamer Ergebnisse zu verabreichen, und bei oraler Applikation beträgt die Dosierung etwa 0.01 bis 25 mg/kg, vorzugsweise 0.1 bis 10 mg/kg Körpergewicht.

Trotzdem kann es gegebenenfalls erforderlich sein, von den genannten Mengen abzuweichen, und zwar in Abhängigkeit von Körpergewicht, Applikationsweg, individuellem Verhalten gegenüber dem Wirkstoff, Art der Zubereitung und Zeitpunkt bzw. Intervall, zu welchem die Applikation erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation größerer Mengen kann es empfehlenswert sein, diese in mehreren Einzelgaben über den Tag zu verteilen.

Die Prozentangaben in den folgenden Tests und Beispielen sind, sofern nicht anders angegeben, Gewichtsprozente; Teile sind Gewichtsteile. Lösungsmittelverhältnisse, Verdünnungsverhältnisse und Konzentrationsangaben von flüssig/flüssig-Lösungen beziehen sich jeweils auf das Volumen.

### A. Beispiele

### Verwendete Abkürzungen:

- BINAP: 2,2'-Bis-(diphenylphosphino)-1,1'-binaphthyl
- CDCl₃: Deuterochloroform
- CD₃CN: Deuteroacetonitril
- DC: Dünnschichtchromatographie
- DCI: direkte chemische Ionisation (bei MS)
- DCM: Dichlormethan
- DIEA: N,N-Diisopropylethylamin (Hünig Base)
- DMSO: Dimethylsulfoxid
- DMF: N,N-Dimethylformamid
- d. Th.: der Theorie
- EE: Ethylacetat (Essigsäureethylester)
- EI: Elektronenstoß-Ionisation (bei MS)
- ESI: Elektrospray-Ionisation (bei MS)
- Fp.: Schmelzpunkt
- ges.: gesättigt
- H: Stunde
- HPLC: Hochdruck-, Hochleistungsflüssigchromatographie
- konz.: konzentriert
- LC-MS: Flüssigchromatographie-gekoppelte Massenspektroskopie
- LDA: Lithium-Diisopropylamid
- MS: Massenspektroskopie
- NMR: Kernresonanzspektroskopie
- proz.: prozentig
- RP-HPLC: Reverse Phase HPLC
- RT: Raumtemperatur
- Rₜ: Retentionszeit (bei HPLC)
- THF: Tetrahydrofuran

### Allgemeine LCMS- und HPLC-Methoden:

**Methode 1 (HPLC):** Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0.0 min 2%B, 0.5 min 2%B, 4.5 min 90%B, 6.5 min 90%B; Fluss: 0.75 ml/min; Ofen: 30°C; UV-Detektion: 210 nm.
**Methode 2 (HPLC, Enantiomerentrennung):** Chiraler Kieselgelselektor KBD 6136 (10 µm, 350 x 30 mm) basierend auf dem Selektor Poly(N-metbacryloyl-L-leucin-l-menthylamid); Temperatur: 24°C; Fluss: 50 ml/min; UV-Detektion: 254 nm; Probenaufgabe in Ethylacetat; Elutionsgemische aus iso-Hexan (A)/Ethylacetat (B), z. B.: Gradient: → 0.0 min 40%B → 9.0 min 40%B → 9.01 min 100%B → 12.0 min 100%B → 12.01 min 40%/B → 15 min 40%B.
**Methode 3 (LCMS):** Gerätetyp MS: Micromass ZQ; Gerätetyp HPLC: HP 1100 Series; UV DAD; Säule: Phenomenex Synergi 2µ Hydro-RP Mercury 20 mm x 4 mm; Eluent A: 11 Wasser + 0.5 ml 50%ige Ameisensäure, Eluent B: 1 1 Acetonitril + 0.5 ml 50 %ige Ameisensäure; Gradient: 0.0 min 90 %A → 2.5 min 30 %A → 3.0 min 5 %A → 4.5 min 5 %A; Fluss: 0.0 min 1 ml/min, 2.5 min/3.0 min/4.5 min 2 ml/min; Ofen: 50°C; UV-Detektion: 210 nm.
**Methode 4 (HPLC, präparative Trennung):** Säule: CromSil C18, 250 mm x 30 mm; Eluent A: Wasser, Eluent B: Acetonitril; Gradient: 3 min 10%B -> 31 min 90%B -> 34 min 90%B -> 34.01 min 10%B; Laufzeit: 38 min; Fluss: 50 ml/min; UV-Detektion: 210 nm.
**Methode 5 (LCMS):** Gerätetyp MS: Micromass ZQ; Gerätetyp HPLC: Waters Alliance 2795; Säule: Phenomenex Synergi 2µ Hydro-RP Mercury 20 mm x 4 mm; Eluent A: 11 Wasser + 0.5 ml 50 %ige Ameisensäure, Eluent B: 1 1 Acetonitril + 0.5 ml 50 %ige Ameisensäure; Gradient: 0.0 min 90 %A → 2.5 min 30 %A → 3.0 min 5 %A → 4.5 min 5 %A; Fluss: 0.0 min 1 ml/min, 2.5 min/3.0 min/4.5 min 2 ml/min; Ofen: 50°C; UV-Detektion: 210 nm.
**Methode 6 (HPLC):** Instrument: HP 1100 mit DAD-Detektion; Säule: Kromasil RP-18, 60 mm x 2 mm, 3.5 µm; Eluent A: 5 ml HClO₄/l Wasser, Eluent B: Acetonitril; Gradient: 0.0 min 2 %B, 0.5 min 2 %B, 4.5 min 90 %B, 9 min 90 %B; Fluss: 0.75 ml/min; Ofen: 30°C; UV-Detektion: 210 nm.
**Methode 7 (LC-MS):** Instrument: Micromass Platform LCZ mit HPLC Agilent Serie 1100; Säule: Grom-SIL120 ODS-4 HE, 50 mm x 2.0 mm, 3 µm; Eluent A: 1 1 Wasser + 1 ml 50 %ige Ameisensäure, Eluent B: 1 1 Acetonitril + 1 ml 50 %ige Ameisensäure; Gradient: 0.0 min 100 %A → 0.2 min 100 %A → 2.9 min 30 %A → 3.1 min 10 %A → 4.5 min 10 %A; Ofen: 55°C; Fluss: 0.8 ml/min; UV-Detektion: 210 nm.

### Ausführungsbeispiele

### Allgemeine Arbeitsvorschrift [A]: Synthese von substituierten 2-Aminozimtsäurederivaten mittels Heck-Kupplung aus 2-halogensubstituierten Anilinen

In einem Einhalskolben werden 1.0 Äquivalente eines Arylhalogenids mit 1.6 Äquivalenten Acrylsäuremethylester, 2.0 Äquivalenten Triethylamin, 0.03 Äquivalenten Palladium(II)acetat und 0.03 Äquivalenten Tri-o-tolylphosphin in Acetonitril vorgelegt (ca. 1M-Lösung). Man lässt das Gemisch unter Rückfluss für 48 Stunden rühren. Nach beendeter Reaktion (Reaktionskontrolle mittels DC) wird das Lösemittel entfernt. Der Rückstand wird über Kieselgel mit Cyclohexan/Ethylacetat = 8:2 v/v chromatographisch gereinigt.

### Beispiel 1A

### (2E)-3-[2-Amino-3-fluorphenyl]-propensäuremethylester

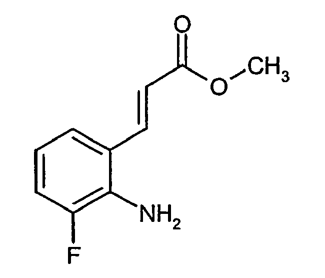

Ausgehend von 42.00 g (221.04 mmol) 2-Brom-6-fluoranilin werden nach der allgemeinen Arbeitsvorschrift [A] 29.66 g (68% d. Th.) Produkt erhalten.
HPLC (Methode 1): Rₜ = 4.14 min
MS (ESI-pos): m/z = 196 (M+H)⁺

### Beispiel 2A

### 2-Amino-3-[(1E)-3-methoxy-3-oxo-1-propenyl]benzoesäuremethylester

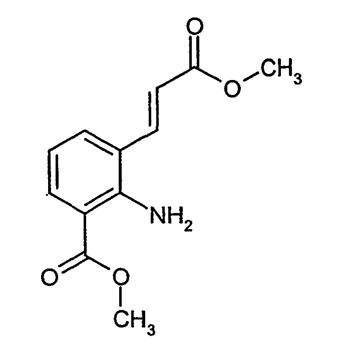

Ausgehend von 2.00 g (8.69 mmol) 2-Amino-3-brombenzoesäuremethylester werden nach der allgemeinen Arbeitsvorschrift [A] 1.29 g (60% d. Th.) Produkt erhalten.
HPLC (Methode 1): Rₜ= 4.42 min
MS (ESI-pos): m/z = 236 (M+H)⁺

### Beispiel 3A

### (2E)-3-(2-Amino-3,5-difluorphenyl)-2-propensäuremethylester

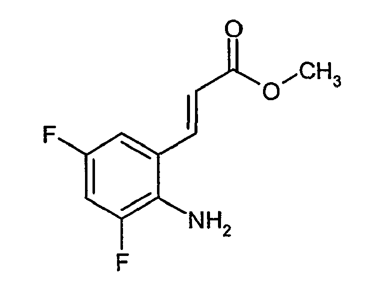

Ausgehend von 3.00 g (14.42 mmol) 2-Brom-4,6-difluoranilin werden nach der allgemeinen Arbeitsvorschrift [A] 1.41 g (45% d. Th.) Produkt erhalten.
HPLC (Methode 1): Rₜ= 4.23 min
MS (ESI-pos): m/z = 214 (M+H)⁺

### Beispiel 4A

### 4-Amino-3-[(1E)-3-methoxy-3-oxo-1-propenyl]benzoesäuremethylester

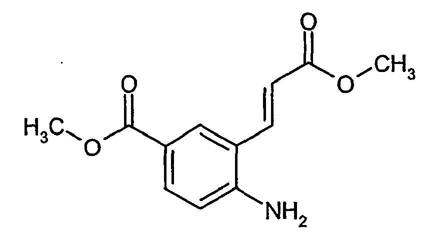

Ausgehend von 25.00 g (90.23 mmol) 4-Amino-3-iod-benzoesäuremethylester werden nach der allgemeinen Arbeitsvorschrift [A] 24.31 g (92% d. Th.) Produkt erhalten.
HPLC (Methode 1): Rₜ= 4.71 min
MS (ESI-pos): m/z = 278 (M+H)⁺

### Beispiel 5A

### (2E)-3-[2-Amino-5-cyanophenyl]-2-propensäuremethylester

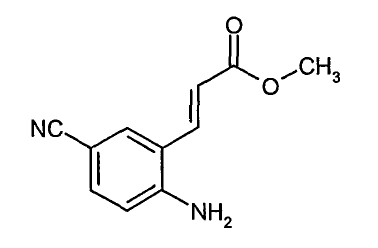

Ausgehend von 1.90 g (9.64 mmol) 3-Brom-4-aminobenzonitril werden nach der allgemeinen Arbeitsvorschrift [A] 1.28 g (50% d. Th.) Produkt erhalten.
HPLC (Methode 1): Rₜ= 2.85 min
MS (DCI-pos): m/z = 220 (M+NH₄)⁺

### Allgemeine Arbeitsvorschrift [B]: Synthese von substituerten 2-Nitrozimtsäure-Derivaten mittels Wittig-Horner-Reaktion aus 2-halogensubstituierten Benzaldehyden

In einem 100 ml Einhalskolben werden 27.5 mmol Methyldiethylphosphonacetat, 25.0 mmol des Benzaldehyds mit 27.5 mmol Lithiumhydroxid in Tetrahydrofuran suspendiert. Nach beendeter Reaktion (Reaktionskontrolle mittels DC) wird der Ansatz mit gleichem Volumen Wasser versetzt. Man extrahiert die wässrige Phase dreimal mit Ethylacetat. Die vereinigten organischen Phasen werden mit gesättigter Natriumchlorid-Lösung gewaschen, über Natriumsulfat getrocknet und das Lösemittel entfernt. Das Produkt wird im Hochvakuum bei RT ohne weitere Reinigung getrocknet. Gegebenenfalls wird bei starker Verunreinigung säulenchromatographisch über Kieselgel mit Cyclohexan/Ethylacetat gereinigt.

### Beispiel 6A

### (2E)-3-(3-Methoxy-2-nitrophenyl)-2-propensäuremethylester

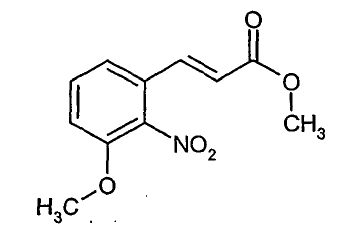

Ausgehend von 2.00 g (11.04 mmol) 3-Methoxy-2-nitrobenzaldehyd werden nach der allgemeinen Arbeitsvorschrift [B] 2.46 g (92% d. Th.) Produkt erhalten.
HPLC (Methode 1): Rₜ = 4.37 min
MS (ESI-pos): m/z = 238 (M+H)⁺

### Beispiel 7A

### (2E)-3-(5-Fluor-2-nitrophenyl)-2-propensäuremethylester

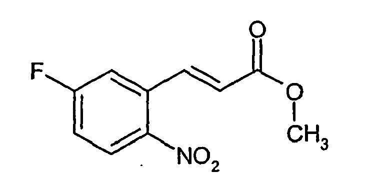

Ausgehend von 20.0 g (118.3 mmol) 5-Fluor-2-nitrobenzaldehyd werden nach der allgemeinen Arbeitsvorschrift [B] 7.25 g (27% d. Th.) Produkt erhalten.
MS (DCI): m/z = 243 (M+NH₄)⁺

### Allgemeine Arbeitsvorschrift [C]: Herstellung eines 2-Nitrobezaldehyds aus einem Benzylhalogenid

10.0 mmol des Benzylhalogenids werden mit 4.1 g Molekularsieb 4Å und 20.0 mmol *N*-Methylmorpholin-*N*-Oxid in 45 ml Acetonitril suspendiert. Man lässt bis zur Umsetzung (Reaktionskontrolle mittels DC) bei RT rühren. Nach beendeter Reaktion wird das Molekularsieb abfiltriert, das Lösungsmittel eingeengt und der Rückstand wieder in Ethylacetat aufgenommen. Diese Lösung wird zunächst mit 1N Salzsäure gewaschen und dann mit gesättigter Natriumchlorid-Lösung. Die abgetrennte organische Phase läßt man dann über Natriumsulfat trocknen und engt das Lösungsmittel wieder ein. Das Rohprodukt verfügt laut Analytik über eine genügend hohe Reinheit und kann direkt weiter umgesetzt werden.

### Beispiel 8A

### 2-Fluor-6-nitrobenzaldehyd

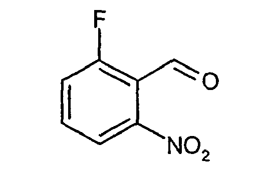

Ausgehend von 2.00 g (8.55 mmol) 3-Fluor-6-nitrobenzylbromid werden nach der allgemeinen Arbeitsvorschrift [C] 1.09 g (75% d. Th.) Produkt erhalten.
HPLC (Methode 1): Rₜ= 3.58 min

### Allgemeine Arbeitsvorschrift [D]: Reduktion der Nitrogruppe der 2-Nitrozimtsäurederivate

In einem 250 ml Zweihalskolben werden unter Argon in 60 ml absolutem Ethanol oder Methanol 25 mmol der Nitroverbindung und 125 mmol Zinn-II-chloriddihydrat vorgelegt. Diese Suspension wird 30 Minuten unter Rückfluss gerührt, und es entsteht eine klare Lösung. Dann lässt man die Lösung auf Raumtemperatur abkühlen und gießt sie danach auf Eiswasser. Der pH-Wert wird entweder mit festem Natriumhydrogencarbonat oder mit einer gesättigten Natriumcarbonat-Lösung auf pH=7-8 eingestellt. Jetzt gibt man 60 ml Ethylacetat hinzu und filtriert die ausgefallenen Zinnsalze über Kieselgur (ca. 1 cm Schichtdicke) ab. Die organische Phase wird abgetrennt, und die wässrige Phase wird noch einmal mit Ethylacetat extrahiert. Man vereinigt die organischen Phasen und wäscht sie einmal mit gesättigter Natriumchlorid-Lösung, trocknet sie über Natriumsulfat und engt das Lösemittel ca. um die Hälfte ein. Nun fügt man Aktivkohle hinzu, entsprechend 1% des Gewichts der Nitroverbindung, und erhitzt für 30 Minuten unter Rückfluss (Verfärbung der Lösung). Die Aktivkohle wird abfiltriert und das Lösemittel eingeengt.

Als Rückstand verbleibt ein Öl, das bei Trocknung bei RT im Hochvakuum Kristalle ausbildet. Ohne weitere Aufreinigung erfolgt eine direkte Umsetzung zur nächsten Stufe.

### Beispiel 9A

### 3-[2-Amino-6-fluorphenyl]-propensäuremethylester

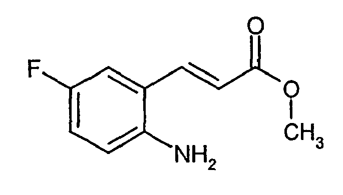

Ausgehend von 7.25 g (32.2 mmol) Nitroverbindung aus Beispiel 7A werden nach der allgemeinen Arbeitsvorschrift [D] 5.0 g (58% d. Th.) Produkt erhalten.
HPLC (Methode 1): Rₜ= 3.33 min

### Allgemeine Arbeitsvorschrift [E]: Synthese der N-Arylpiperidin-4-carbonsäureester mittels Buehwald-Hartwig-Chemie

Die Reaktion wird unter Argon in einem gründlich ausgeheizten Kolben durchgeführt. In 100 ml absolutem Toluol werden 24.7 mmol des Brombenzols, 74 mmol Piperidin-4-carbonsäureester sowie 34.5 mmol Natrium-tert.-butylat vorgelegt, 0.25 mmol Tris(dibenzylidenaceton)dipalladium und 0.74 mmol BINAP zugegeben, das Reaktionsgemisch auf 120°C erwärmt und 16 Stunden unter Rückfluss erhitzt. Der Ansatz wird abgebrochen und nacheinander einmal mit Wasser und zweimal mit 1N Salzsäure extrahiert. Anschließend wird die saure wässerige Phase mit 1N Natronlauge auf pH 8 eingestellt und dreimal mit Dichlormethan extrahiert. Die vereinigten organischen Phasen werden über Natriumsulfat getrocknet, filtriert und das Lösungsmittel im Vakuum entfernt. Das so erhaltene Rohprodukt kann ohne weitere Reinigung direkt weiter umgesetzt werden.

### Beispiel 10A

### N-(4-Fluor-3-methylphenyl)-piperidin-4-carbonsäuremethylester

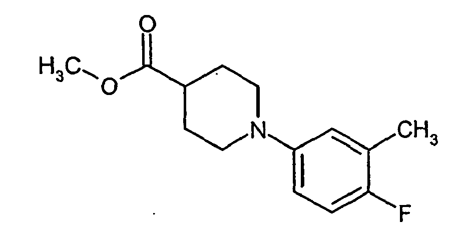

Ausgehend von 10.6 g (74.0 mmol) Piperidin-4-carbonsäuremethylester und 4.67 g (24.7 mmol) 5-Brom-2-fluortoluol werden nach der allgemeinen Arbeitsvorschrift [E] 2.74 g (40% d. Th.) Produkt erhalten.
HPLC (Methode 1): Rₜ= 3.48 min

Die Beispiele 11A bis 17A aus der nachfolgenden Tabelle können nach der allgemeinen Arbeitsvorschrift [E] hergestellt werden.

| **Bsp.-Nr.** | **Struktur** | **Edukt A Menge Edukt A Edukt B Menge Edukt B** | **Ausbeute** | **HPLC Rₜ [min] (Methode)** |
|---|---|---|---|---|
| **11A** | 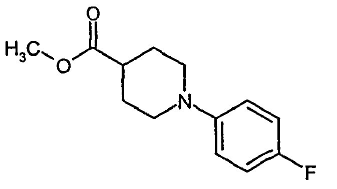 | Piperidin-4-carbon-säuremethylester 10.6 g (74.0 mmol) 4-Fluor-brombenzol 4.32 g (24.7 mmol) | 5.77 g (62% d. Th.) | 3.25 (1) |
| **12A** | 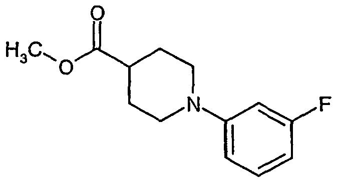 | Piperidin-4-carbon-säuremethylester 10.6 g (74.0 mmol) 3-Fluor-brombenzol 4.32 g (24.7 mmol) | 3.19 g (54% d. Th.) | 3.41 (1) |
| **13A** | 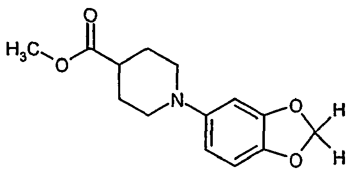 | Piperidin-4-carbon-säuremethylester 10.6 g (74.0 mmol) 3,4-Dioxolan-brom-benzol 4.96 g (24.7 mmol) | 3.75 g (44% d. Th.) | 3.27 (1) |
| **14A** | 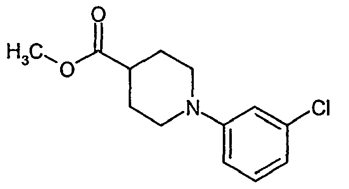 | Piperidin-4-carbon-säuremethylester 22.4 g (156.7 mmol) 3-Chlor-brombenzol 10.00 g (52.3 mmol) | 6.78 g (51% d. Th.) | 3.74 (1) |
| **15A** | 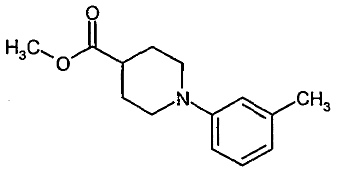 | Piperidin-4-carbon-säuremethylester 5.02 g (35.1 mmol) 3-Methylbrombenzol 2.00 g (11.7 mmol) | 0.59 g (9% d. Th.) | 3.46 (1) |
| **16A** | 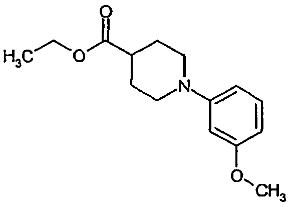 | Piperidin-4-carbonsäure-ethylester 5.00 g (31.8 mmol) 3-Methoxy-brombenzol 1.98 g (10.6 mmol) | 1.66 g (59% d. Th.) | 3.60 (1) |
| **17A** | 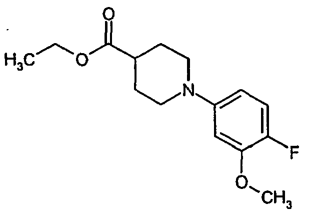 | Piperidin-4-carbonsäure-ethylester 3.68 g (23.41 mmol) 4-Brom-1-fluor-2-methoxybenzol 1.60 g (7.80 mmol) | 1.32 g (60% d. Th.) | 3.60 (1) |

### Allgemeine Arbeitsvorschrift [F]: Hydrolyse der N-Arylpiperidin-4-carbonsäureester

Es werden 1.0 Äquivalente des N-Arylpiperidin-4-carbonsäureesters in Dioxan gelöst und 2.0 Äquivalente 1N Natronlauge hinzugefügt. Man läßt für 16 Stunden bei 80°C rühren und nach beendeter Reaktion (Reaktionskontrolle mittels analytischer HPLC) wird der Ansatz eingeengt. Der Rückstand wird dann in Wasser aufgenommen und mit 1N Salzsäure auf pH = 5 gestellt. Man filtriert den entstehenden Niederschlag ab, wäscht ihn mit wenig Wasser sowie Cyclohexan und trocknet ihn im Hochvakuum bei Raumtemperatur. Falls die Reinheit des Produktes nicht hoch genug ist, wird es über präparative HPLC an RP-Phase gereinigt.

### Beispiel 18A

### N-(4-Fluor-3-methylphenyl)-piperidin-4-carbonsäure

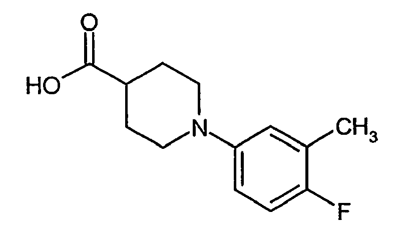

Ausgehend von 2.57 g (10.7 mmol) des Esters aus Beispiel 10A werden nach der allgemeinen Arbeitsvorschrift [F] 1.48 g (58% d. Th.) Produkt erhalten.
HPLC (Methode 1): Rₜ= 3.35 min

Die Beispiele 19A bis 25A aus der nachfolgenden Tabelle können nach der allgemeinen Arbeitsvorschrift [F] hergestellt werden.

| **Bsp.-Nr.** | **Struktur** | **Edukt Menge Edukt** | **Ausbeute** | **HPLC Rₜ [min] (Methode)** |
|---|---|---|---|---|
| **19A** | 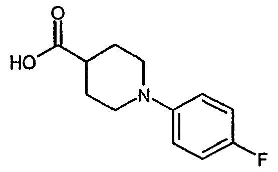 | Beispiel 11A 5.7 g (24.0 mmol) | 2.06 g (38% d. Th.) | 2.77 (1) |
| **20A** | 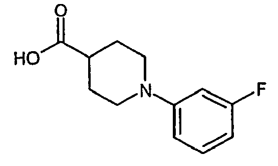 | Beispiel 12A 3.19 g (13.4 mmol) | 2.5 g (83% d. Th.) | 3.05 (1) |
| **21A** | 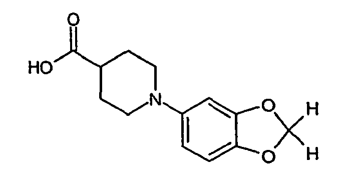 | Beispiel 13A 3.75 g (14.2 mmol) | 2.85 g (80% d. Th.) | 2.98 (1) |
| **22A** | 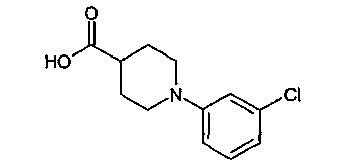 | Beispiel 14A 6.73 g (26.5 mmol) | 5.55 g (76% d. Th.) | 3.36 (1) |
| **23A** | 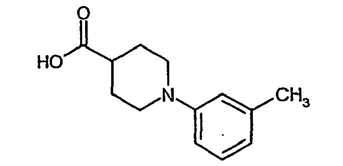 | Beispiel 15A 0.59 g (1.09 mmol) | 0.13 g (48% d. Th.) | 1.13 (3) |
| **24A** | 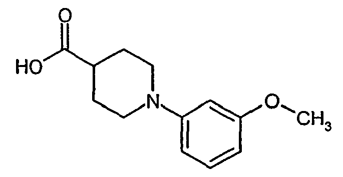 | Beispiel 16A 1.57 g (5.96 mmol) | 1.12 g (80% d. Th.) | 3.10 (1) |
| **25A** | 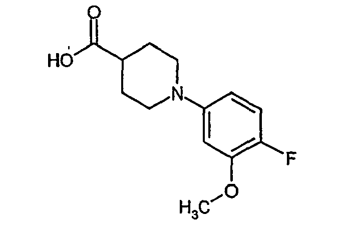 | Beispiel 17A 5.72 g (20.33 mmol) | 4.98 g (97% d. Th.) | 3.20 (1) |

### Allgemeine Arbeitsvorschrift [G]: Acylierung der 2-Aminozimtsäureester mit N-Arylpiperidin-4-carbonsäure

In 75 ml Dichlormethan werden 6.3 mmol der N-Arylpiperidin-4-carbonsäure vorgelegt, ein Tropfen DMF zugegeben und das Gemisch unter Eiskühlung mit 18.7 mmol Oxalylchlorid versetzt. Nach vollständiger Zugabe wird 1h unter Rückfluss erhitzt, das Reaktionsgemisch abgekühlt und das Lösungsmittel im Vakuum entfernt. Der Rückstand wird in 60 ml Dichlormethan aufgenommen und unter Eiskühlung zu einer Lösung von 5.7 mmol 2-Aminozimtsäureester und 18.7 mmol Pyridin oder Triethylamin in 20 ml Dichlormethan zugetropft. Nach vollständiger Zugabe wird für 2h unter Rückfluss erhitzt. Anschließend wird das Lösungsmittel im Vakuum entfernt und der Rückstand in Dichlormethan aufgenommen. Die organische Phase wird zweimal mit Wasser und einmal mit gesättigter Natriumhydrogencarbonatlösung extrahiert und über Natriumsulfat getrocknet. Das Natriumsulfat wird abfiltriert und das Lösungsmittel im Vakuum entfernt. Der Rückstand wird mit Diethylether und einigen Tropfen Ethylacetat verrührt, der Niederschlag abfiltriert und mit Diethylether nachgewaschen. Das Produkt wird im Vakuum getrocknet. Sollte das Produkt nicht sauber genug für weitere Umsetzungen sein, wird es chromatographisch gereinigt.

### Beispiel 26A

### (2E)-3-Fluor-2-({[1-(4-fluor-3-methyl)piperidin-4-yl]carbonyl}amino)phenyl]acrylsäure-methylester

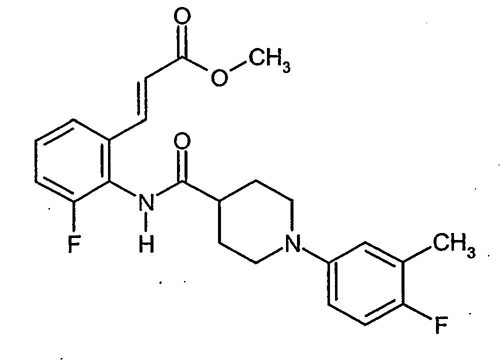

Ausgehend von 1.48 g (6.23 mmol) Carbonsäure aus Beispiel 18A und 1.11 g (5.67 mmol) Anilin aus Beispiel 1A werden nach der allgemeinen Arbeitsvorschrift [G] 7.77 g (79% d. Th.) Produkt erhalten.
HPLC (Methode 1): Rₜ= 4.05 min

Die Beispiele 27A bis 33A aus der nachfolgenden Tabelle können nach der allgemeinen Arbeitsvorschrift [G] hergestellt werden.

| **Bsp.-Nr.** | **Struktur** | **Anilin Menge Anilin Säure Menge Säure** | **Ausbeute** | **HPLC Rₜ [min] (Methode)** |
|---|---|---|---|---|
| **27A** | 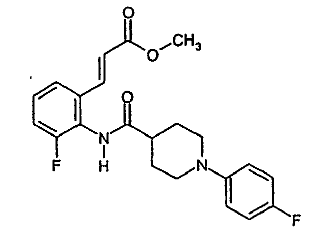 | Beispiel 1A 4.81 g (24.67 mmol) Beispiel 19A 5.8 g (25.98 mmol) | 4.70 g (48% d. Th.) | 3.80 (6) |
| **28A** | 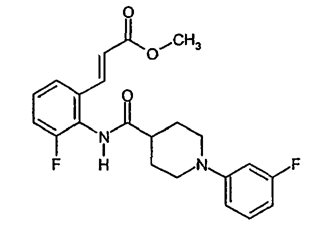 | Beispiel 1A 1.99 g (10.2 mmol) Beispiel 20A 2.5 g (11.2 mmol) | 3.75 g (92% d. Th.) | 3.97 (1) |
| **29A** | 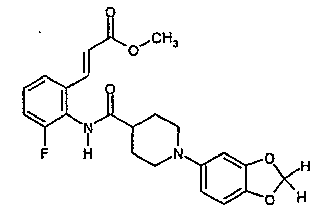 | Beispiel 1A 2.03 g (10.4 mmol) Beispiel 21A 2,85 g (11.4 mmol) | 1.79 g (39% d. Th.) | 3.89 (1) |
| **30A** | 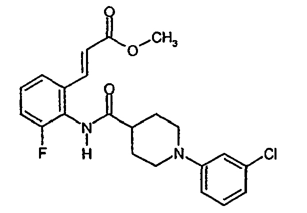 | Beispiel 1A 1.9 g (9.9 mmol) Beispiel 22A 3.0 g (10.9 mmol) | 2.78 g (68% d. Th.) | 4.20 (1) |
| **31A** | 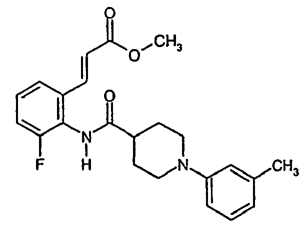 | Beispiel 1A 0.22 g (1.11 mmol) Beispiel 23A 0.29 g (1.20 mmol) | 0.21 g (46% d. Th.) | 3.99 (6) |
| **32A** | 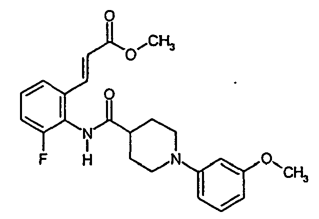 | Beispiel 1A 0.87 g (4.45 mmol) Beispiel 24A 1.1 g (4.68 mmol) | 0.47 g (26% d. Th.) | 4.00 (1) |
| **33A** | 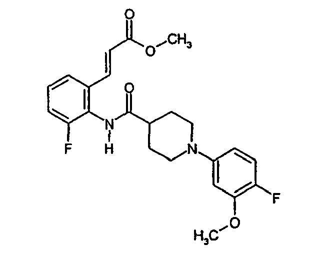 | Beispiel 1A 0.73 g (3.76 mmol) Beispiel 25A 2.40 g (9.48 mmol) | 0.25 g (15% d. Th.) | 4.00 (1) |

### Allgemeine Arbeitsvorschrift [H]: Cyclisierung der 2-Aminoacylzimtsäureester mit Anilinen

In 10 ml Toluol werden bei Raumtemperatur 1.2 mmol des 2-Aminoacylzimtsäureesters sowie 7.24 mmol Phosphoroxychlorid vorgelegt. Das Gemisch wird unter intensivem Rühren 16h unter Rückfluss erhitzt (Badtemperatur 120-125°C). Anschließend wird das Lösungsmittel im Vakuum abdestilliert und einmal mit Toluol codestilliert. Es wird erneut in 10 ml Toluol aufgenommen und mit 3.6 mmol des Anilins versetzt. Das Gemisch wird 24h bei Rückfluss erhitzt. Anschließend wird das Lösungsmittel im Vakuum entfernt und der Rückstand mit Dichlormethan aufgenommen und zweimal mit 1N Salzsäure extrahiert. Die organische Phase wird über Natriumsulfat getrocknet, filtriert und das Lösungsmittel im Vakuum entfernt. Das Produkt wird chromatographisch an Kieselgel oder mittels präparativer HPLC (Methode 4) gereinigt.

### Beispiel 34A

### {8-Fluor-2-[1-(4-fluor-3-methylphenyl)piperidin-4-yl]-3-[2-methoxy-5-(trifluormethyl)phenyl]-3,4-dihydroquinazolin-4-yl}-essigsäuremethylester

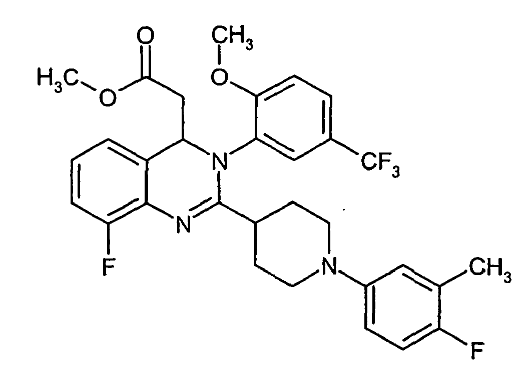

Ausgehend von 0.6 g (1.45 mmol) 2-Acylaminozimtsäureester aus Beispiel 26A werden nach der allgemeinen Arbeitsvorschrift [H] und Reinigung mittels präparativer HPLC (Methode 4) sowie Chromatographie an Kieselgel mit Cyclohexan/Ethylacetat 8:2 (v/v) 355 mg (39% d. Th.) Produkt erhalten.
HPLC (Methode 1): Rₜ= 4.52 min
MS: m/z = 588 (M+H)⁺

### Beispiel 35A

### {8-Fluor-2-[1-(4-fluor-3-methylphenyl)piperidin-4-yl]-3-[2-methoxy-5-(trifluormethyl)phenyl]-3,4-dihydroquinazolin-4-yl}-essigsäuremethylester

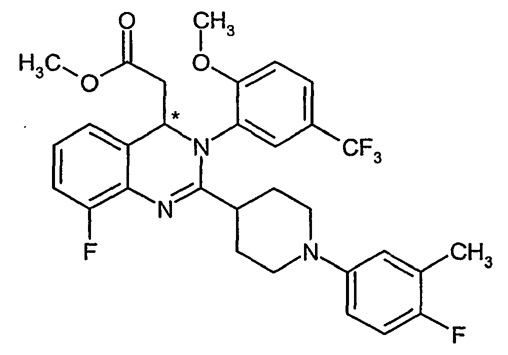

Ausgehend von 355 mg (0.6 mmol) Racemat aus Beispiel 34A werden nach chromatographischer Enantiomerentrennung (Methode 2) 148 mg (42% d. Th.) der Verbindung als Enantiomer B erhalten.

### Beispiel 36A

### {8-Fluor-2-[1-(4-fluorphenyl)piperidin-4-yl]-3-[2-methoxy-5-(trifluormethyl)phenyl]-3,4-dihydro-quinazolin-4-yl}-essigsäuremethylester

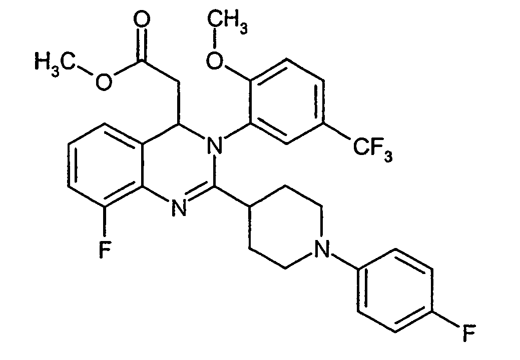

Ausgehend von 300 mg (0.75 mmol) 2-Acylaminozimtsäureester aus Beispiel 27A werden nach der allgemeinen Arbeitsvorschrift [H] 298 mg (69% d. Th.) Produkt erhalten.
HPLC (Methode 1): Rₜ= 4.30 min
MS (ESI pos): m/z = 574 (M+H)⁺

### Beispiel 37A

### {8-Fluor-3-(2-methoxy-5-(trifluormethyl)phenyl)-2-[1-(3-methylphenyl)piperidin-4-yl]-3,4-dihydroquinazolin-4-yl}- essigsäuremethylester

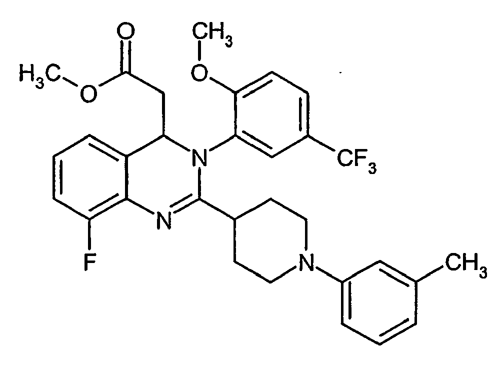

Ausgehend von 0.057 g (0.14 mmol) 2-Acylaminozimtsäureester aus Beispiel 31A werden nach der allgemeinen Arbeitsvorschrift [H] und Reinigung mittels präparativer HPLC (Methode 4) 32 mg (35% d. Th.) Produkt erhalten.
HPLC (Methode 1): Rₜ = 4.44 min
MS: m/z = 570 (M+H)⁺

### Ausführungsbeispiele

### Allgemeine Arbeitsvorschrift [I]: Esterverseifung der Chinazolylessigsäureester

Es werden 1.0 Äquivalente des Chinazolylessigsäureesters in Dioxan gelöst und 5.0 Äquivalente 1N Natronlauge hinzugefügt. Man lässt für 16 Stunden bei 80°C rühren und nach beendeter Reaktion (Reaktionskontrolle mittels analytischer HPLC) wird der Ansatz eingeengt. Der Rückstand wird dann in Wasser aufgenommen und mit 1N Salzsäure auf pH = 5 gestellt. Man filtriert den entstehenden Niederschlag ab, wäscht ihn mit wenig Wasser und Diethylether nach und trocknet ihn im Hochvakuum oder im Trockenschrank. Falls die Reinheit des Produktes nicht hoch genug ist, wird es über präparative HPLC an RP-Phase (Methode 4) oder durch Chromatographie an Kieselgel gereinigt.

### Beispiel 1

### {8-Fluor-2-[1-(4-fluor-3-methylphenyl)piperidin-4-yl]-3-[2-methoxy-5-(trifluorinethyl)phenyl]-3,4-dihydroquinazolin-4-yl}essigsäure Hydrochlorid

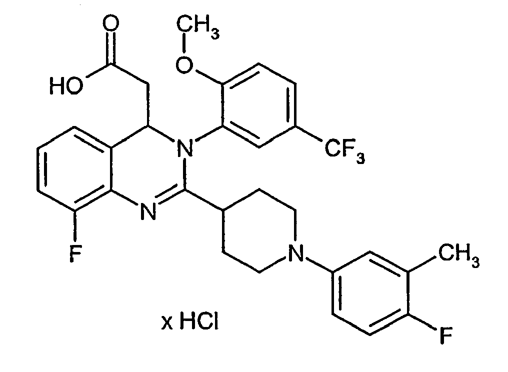

Ausgehend von 50 mg (0.085 mmol) Methylester aus Beispiel 34A werden nach der allgemeinen Arbeitsvorschrift [I] sowie Einengen des Produktes aus einem Gemisch aus Methanol / 1N Salzsäure 13 mg (24% d. Th.) Produkt erhalten.
HPLC (Methode 1): Rₜ= 4.27 min
MS (ESIpos): m/z = 574 (M+H)⁺
¹H-NMR (400MHz, CD₃CN): δ [ppm] = 8.05-7.82 (m, 2H), 7.65-7.38 (m, 3H), 7.30 (d, 1H), 7.29-7.12 (m, 2H), 7.00 (d, 1H), 5.40 und 5.25 (2s, 1H), 4.00-3.75 (2s, 3H), 3.75-2.70 (m, 9H), 2.70-2.00 (m, 2H). (Im ¹H-NMR gibt es Hinweise auf Rotationsisomere.)

### Beispiel 2

### {8-Fluor-2-[1-(4-fluor-3-methylphenyl)piperidin-4-yl]-3-[2-methoxy-5-(trifluormethyl)phenyl]-3,4-dihydroquinazolin-4-yl}essigsäure Hydrochlorid

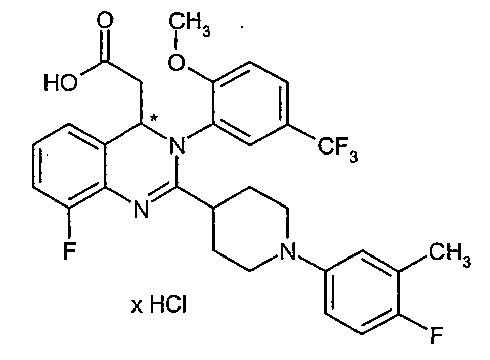

Ausgehend von 148 mg (0.25 mmol) Methylester aus Beispiel 35A werden nach der allgemeinen Arbeitsvorschrift [I] sowie Einengen des Produktes aus einem Gemisch aus Methanol / 1N Salzsäure 62 mg (40% d. Th.) Produkt erhalten.
HPLC (Methode 1): Rₜ= 4.27 min
MS (ESIpos): m/z = 574 (M-HCl+H)⁺
¹H-NMR (400MHz, CD₃CN): δ [ppm] = 8.00-7.55 (m, 2H), 7.55-6.75 (m, 7H), 5.25 und 5.05 (2s, 1H), 3.95 und 3.75 (2s, 3H), 3.70-3.48 (d, 2H), 3.05-2.00 (m, 7H). (Im ¹H-NMR gibt es Hinweise auf Rotationsisomere.)

### Beispiel 3

### {8-Fluor-3-[2-methoxy-5-(trifluormethyl)phenyl]-2-[1-(3-methylphenyl)piperidin-4-yl]-3,4-dihydroquinazolin-4-yl}essigsäure

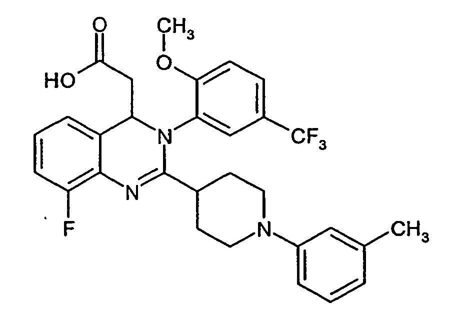

Ausgehend von 41 mg (0.07 mmol) Methylester aus Beispiel 37A werden nach der allgemeinen Arbeitsvorschrift [I] sowie Einengen des Produktes aus einem Gemisch aus Methanol / 1N Salzsäure und anschließender Reinigung mittels Chromatographie (Methode 4) 13 mg (31% d. Th.) Produkt erhalten.
HPLC (Methode 7): Rₜ= 2.64 min
MS (ESIpos): m/z = 556 (M+H)⁺
¹H-NMR (400MHz, D₂O): δ [ppm] = 7.94-7.80 (m ,3H), 7.42-6.93 (m, 7H), 5.45-5.27 (m, 2H), 3.90 (s, 3H), 3.85-3.54 (m, 2H), 3.27-3.02 (m, 2H), 2.96-2.68 (m, 3H), 2.38-1.93 (m, 6H). (Im ¹H-NMR gibt es Hinweise auf Rotationsisomere.)

### Beispiel 4

### {8-Fluor-2-[1-(4-fluorphenyl)piperidin-4-yl]-3-[2-methoxy-5-(trifluormethyl)phenyl]-3,4-dihy-droquinazolin-4-yl} essigsäure

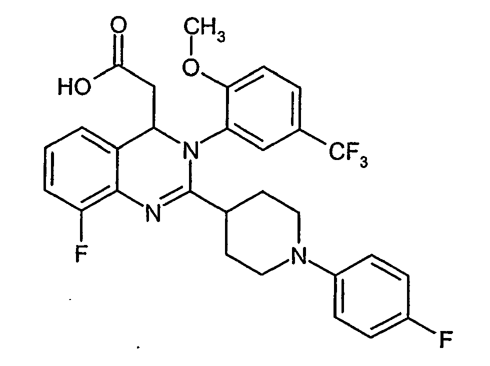

Ausgehend von 50.00 mg (0.09 mmol) Methylester aus Beispiel 36A werden nach der allgemeinen Arbeitsvorschrift [I] 30 mg (62% d. Th.) Produkt erhalten.
HPLC (Methode 1): Rₜ= 4.20 min
MS (DCI/NH₃): m/z = 560 (M+H)⁺
¹H-NMR (400MHz, CDCl₃): δ [ppm] = 7.85-7.50 (m, 2H), 7.35-6.60 (m, 8H), 4.95 (m, 1H), 3.95 und 3.75 (2s, 3H), 3.20-1.50 (m, 10H). (Im ¹H-NMR gibt es Hinweise auf Rotationsisomere.)

Die Beispiele 5 bis 24 aus der nachfolgenden Tabelle können nach den allgemeinen Arbeitsvorschrift [H] und [I] hergestellt werden. Hydrochloride können erhalten werden, indem man gegebenenfalls nach der Umsetzung das Produktes aus einem Gemisch aus Methanol / 1N Salzsäure einengt.

| **Bsp.-Nr.** | **Struktur** | **Molekular-gewicht** | **Isomer** | **HPLC Rₜ [min] (Methode)** | **MS m/z** |
|---|---|---|---|---|---|
| **5** | 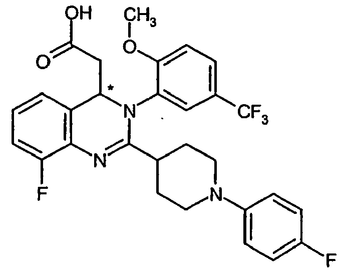 | 559.5 | Enantiomer B | 4.20 (1) | 560 [M+H]⁺ |
| **6** | 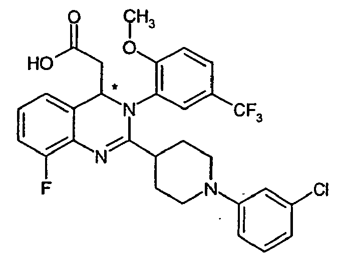 | 576 | Enantiomer B | 4.65 (1) | 576 [M+H]⁺ |
| **7** | 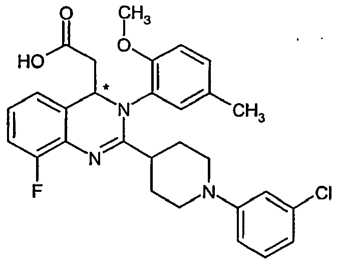 | 522 | Enantiomer B | 4.78 (1) | 522 [M+H]⁺ |
| **8** | 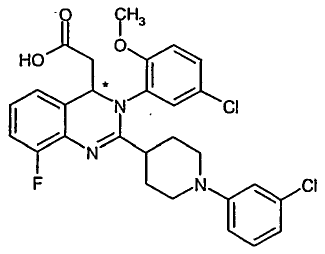 | 542.4 | Enantiomer B | 4.64 (1) | 542 [M+H]⁺ |
| **9** | 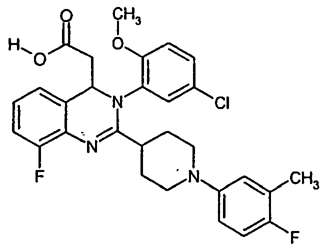 | 540 | Racemat | 4.13 (1) | 540 [M+H]⁺ |
| **10** | 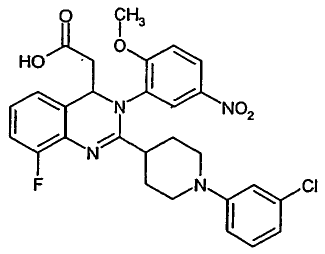 | 553 | Racemat | 4.50 (1) | 553 [M+H]⁺ |
| **11** | 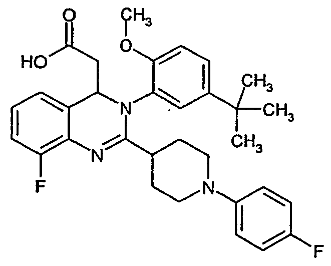 | 547.6 | Racemat | 4.40 (6) | 548 [M+H]⁺ |
| **12** | 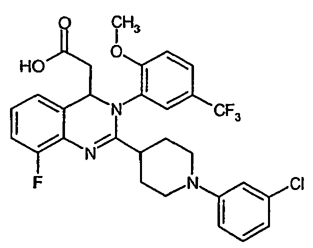 | 576 | Racemat | 4.77 (1) | 576 [M+H]⁺ |
| **13** | 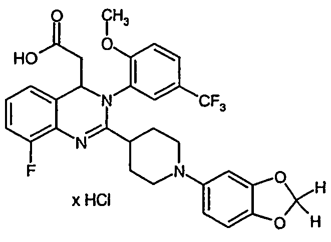 | 588.5 | Racemat | 3.96 (6) | 552 [M-HCl +H]⁺ |
| **14** | 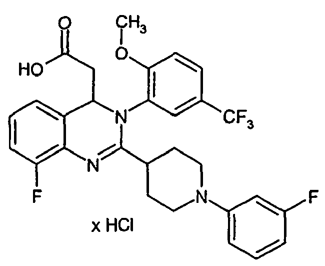 | 596 | Racemat | 4.53 (1) | **m/z** 560 [M-HCl +H]⁺ |
| **15** | 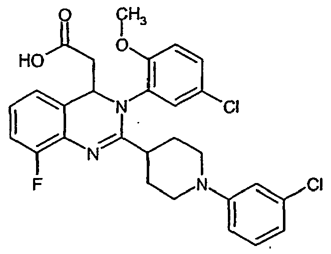 | 542.4 | Racemat | 4.67 (1) | 542 [M+H]⁺ |
| **16** | 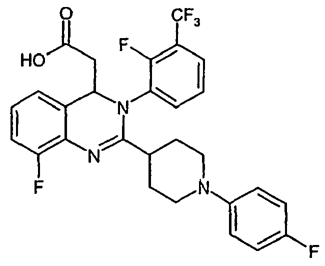 | 547.5 | Racemat | 4.10 (6) | 548 [M+H]⁺ |
| **17** | 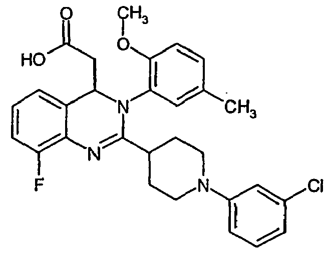 | 522 | Racemat | 4.70 (1) | 522 [M+H]⁺ |
| **18** | 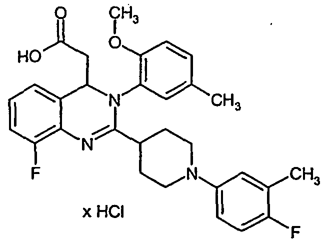 | 556.1 | Racemat | 4.10 (1) | 520 [M-HCl +H]⁺ |
| **19** | 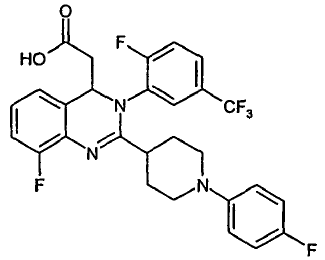 | 547.5 | Racemat | 4.10 (1) | 548 [M+H]⁺ |
| **20** | 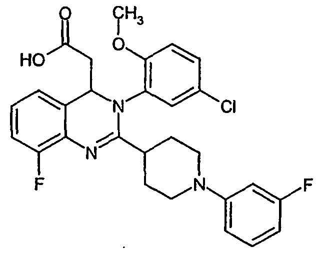 | 562.4 | Racemat | 4.39 (1) | 526 [M-HCl +H]⁺ |
| **21** | 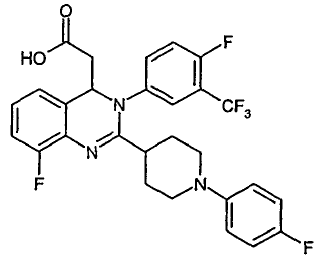 | 547.5 | Racemat | 4.20 (1) | 548 [M+H]⁺ |
| **22** | 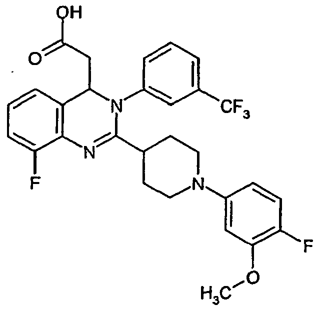 | 559.5 | Racemat | 4.20 (1) | 560 [M+H]⁺ |
| **23** | 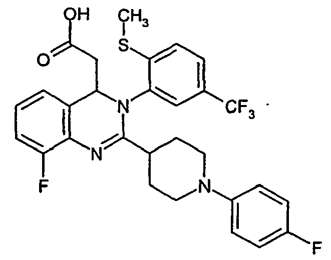 | 575.6 | Racemat | 4.20 (6) | 576 [M+H]⁺ |
| **24** | 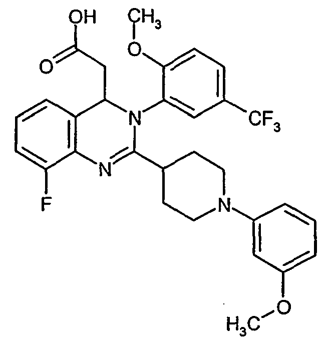 | 571.6 | Racemat | 4.20 (1) | 572 [M+H]⁺ |

### B. Bewertung der physiologischen Wirksamkeit

Die in vitro-Wirkung der erfindungsgemäßen Verbindungen kann in folgenden Assays gezeigt werden:

### Anti-HCMV- (Anti-Humanes Cvtomegalo-Virus) Zytopathogenitätstests

Die Testverbindungen werden als 50 millimolare (mM) Lösungen in Dimethylsulfoxid (DMSO) eingesetzt. Ganciclovir, Foscarnet und Cidofovir dienen als Referenzverbindungen. Nach der Zugabe von jeweils 2 µl der 50, 5, 0.5 und 0.05 mM DMSO-Stammlösungen zu je 98 µl Zellkulturmedium in der Reihe 2 A-H in Doppelbestimmung werden 1:2-Verdünnungen mit je 50 µl Medium bis zur Reihe 11 der 96-Well-Platte durchgeführt. Die Wells in den Reihen 1 und 12 enthalten je 50 µl Medium. In die Wells werden dann je 150 µl einer Suspension von 1 x 104 Zellen (humane Vorhautfibroblasten [NHDF]) pipettiert (Reihe 1 = Zellkontrolle) bzw. in die Reihen 2-12 ein Gemisch von HCMV-infizierten und nichtinfizierten NHDF-Zellen (M.O.I. = 0.001 - 0.002), d.h. 1-2 infizierte Zellen auf 1000 nicht-infizierte Zellen. Die Reihe 12 (ohne Substanz) dient als Viruskontrolle. Die End-Testkonzentrationen liegen bei 250 - 0.0005 µM. Die Platten werden 6 Tage bei 37°C / 5% CO₂ inkubiert, d.h. bis in den Viruskontrollen alle Zellen infiziert sind (100% cytopathogener Effekt [CPE]). Die Wells werden dann durch Zugabe eines Gemisches von Formalin und Giemsa's Farbstoff fixiert und gefärbt (30 Minuten), mit aqua bidest. gewaschen und im Trockenschrank bei 50°C getrocknet. Danach werden die Platten mit einem Overhead-Mikroskop (Plaque multiplier der Firma Technomara) visuell ausgewertet.

Die folgenden Daten können von den Testplatten ermittelt werden:
CC₅₀ (NHDF) = Substanzkonzentration in µM, bei der im Vergleich zur unbehandelten Zellkontrolle keine sichtbaren cytostatischen Effekte auf die Zellen erkennbar sind;
EC₅₀ (HCMV) = Substanzkonzentration in µM, die den CPE (cytopathischen Effekt) um 50 % im Vergleich zur unbehandelten Viruskontrolle hemmt;
SI (Selektivitätsindex) = CC₅₀ (NHDF) / EC₅₀ (HCMV).

Repräsentative in-vitro-Wirkdaten für die erfindungsgemäßen Verbindungen sind in Tabelle A wiedergegeben:

**Tabelle A**

| **Beispiel-Nr.** | **NHDF CC₅₀ [µM]** | **HCMV EC₅₀ [µM]** | **SI HCMV** |
|---|---|---|---|
| **1** | 24 | 0.03 | 800 |
| **2** | 31 | 0.04 | 775 |
| **3** | 31 | 0.1 | 310 |
| **4** | 36 | 0.15 | 237 |

Die Eignung der erfindungsgemäßen Verbindungen zur Behandlung von HCMV-Infektionen kann im folgenden Tiermodell gezeigt werden:

### HCMV Xenograft-Gelfoam^{®}-Modell

**Tiere:** 3-4 Wochen alte weibliche immundefiziente Mäuse (16-18 g), Fox Chase SCID oder Fox Chase SCID-NOD oder SCID-beige werden von kommerziellen Züchtern (Bomholtgaard, Jackson) bezogen. Die Tiere werden unter sterilen Bedingungen (einschließlich Streu und Futter) in Isolatoren gehalten.

**Virusanzucht:** Humanes Cytomegalovirus (HCMV), Stamm Davis, wird in vitro auf humanen embryonalen Vorhautfibroblasten (NHDF-Zellen) angezüchtet. Nach Infektion der NHDF-Zellen mit einer Multiplizität der Infektion (M.O.I) von 0.01 werden die virusinfizierten Zellen 5-7 Tage später geerntet und in Gegenwart von Minimal Essential Medium (MEM), 10% foetalem Kälberserum (FKS) mit 10% DMSO bei -40°C aufbewahrt. Nach serieller Verdünnung der virusinfizierten Zellen in Zehnerschritten erfolgt die Titerbestimmung auf 24-Well-Platten konfluenter NHDF-Zellen nach Vitalfärbung mit Neutralrot oder Fixierung und Färbung mit einem Formalin-Giemsa Gemisch (wie unter B. beschrieben).

**Vorbereitung der Schwämme, Transplantation, Behandlung und Auswertung:** 1x1x1 cm große Kollagenschwämme (Gelfoam^{®}; Fa. Peasel & Lorey, Best.-Nr. 407534; K.T. Chong et al., Abstracts of 39th Interscience Conference on Antimicrobial Agents and Chemotherapy, 1999, S. 439; P.M. Kraemer et al., Cancer Research 1983, (43): 4822-4827) werden zunächst mit Phosphat-gepufferter Saline (PBS) benetzt, die eingeschlossenen Luftblasen durch Entgasen entfernt und dann in MEM + 10 % FKS aufbewahrt. 1 x 106 virusinfizierte NHDF-Zellen (Infektion mit HCMV-M.O.1 = 0.01) weden 3 Stunden nach Infektion abgelöst und in 20 µl MEM, 10% FKS auf einen feuchten Schwamm getropft. Optional werden nach 12-13 Stunden auf die infizier- ten Schwämme 5 ngµl basic Fibroblast Growth Factor (bFGF) in 25 µl PBS / 0.1 % BSA / 1 mM DTT aufgebracht und 1 Stunde inkubiert. Zur Transplantation werden die immundefizienten Mäuse mit Avertin oder einem Gemisch aus Azepromazin-Xylazin und Ketamin narkotisiert, das Rückenfell mit Hilfe eines Trockenrasierers entfernt, die Oberhaut 1-2 cm geöffnet, entlastet und die feuchten Schwämme unter die Rückenhaut transplantiert. Die Operationswunde wird mit Gewebekleber verschlossen. 24 Stunden nach der Transplantation werden die Mäuse über einen Zeitraum von 8 Tagen dreimal täglich (7.00 Uhr und 14.00 Uhr und 19.00 Uhr), zweimal täglich (8.00 Uhr und 17.00 Uhr), oder einmal täglich (14.00 Uhr) peroral mit Substanz behandelt. Die Dosis beträgt 3 oder 10 oder 30 oder 100 mg/kg Körpergewicht, das Applikationsvolumen 10 ml/kg Körpergewicht. Die Formulierung der Substanzen erfolgt in Form einer 0.5%-igen Tylosesuspension optional mit 2 % DMSO. 9 Tage nach Transplantation und 16 Stunden nach der letzten Substanzapplikation werden die Tiere schmerzlos getötet und der Schwamm entnommen. Die virusinfizierten Zellen werden durch Kollagenaseverdau (330 U/1.5 ml) aus dem Schwamm freigesetzt und in Gegenwart von MEM, 10 % foetalem Kälberserum, 10 % DMSO bei -140°C aufbewahrt. Die Auswertung erfolgt nach serieller Verdünnung der virusinfizierten Zellen in Zehnerschritten durch Titerbestimmung auf 24-Well- Platten konfluenter NHDF-Zellen nach Vitalfärbung mit Neutralrot oder nach Fixierung und Färbung mit einem Formalin-Giemsa Gemisch (wie unter B. beschrieben). Ermittelt wird die Anzahl infektiöser Viruspartikel nach Substanzbehandlung im Vergleich zur placebobehandelten Kontrollgruppe.

### C. Ausführungsbeispiele für pharmazeutische Zusammensetzungen

Die erfindungsgemäßen Verbindungen können folgendermaßen in pharmazeutische Zubereitungen überführt werden:

### Tablette:

**Zusammensetzung:** 100 mg der Verbindung von Beispiel 1, 50 mg Lactose (Monohydrat), 50 mg Maisstärke (nativ), 10 mg Polyvinylpyrolidon (PVP 25) (Fa. BASF, Ludwigshafen, Deutschland) und 2 mg Magnesiumstearat.
Tablettengewicht 212 mg. Durchmesser 8 mm, Wölbungsradius 12 mm.
**Herstellung:** Die Mischung aus Wirkstoff, Lactose und Stärke wird mit einer 5%-igen Lösung (m/m) des PVPs in Wasser granuliert. Das Granulat wird nach dem Trocknen mit dem Magnesiumstearat für 5 min. gemischt. Diese Mischung wird mit einer üblichen Tablettenpresse verpresst (Format der Tablette siehe oben). Als Richtwert für die Verpressung wird eine Presskraft von 15 kN verwendet.

### Oral applizierbare Suspension:

**Zusammensetzung:** 1000 mg der Verbindung von Beispiel 1, 1000 mg Ethanol (96%), 400 mg Rhodigel (Xanthan gum der Fa. FMC, Pennsylvania, USA) und 99 g Wasser.

Einer Einzeldosis von 100 mg der erfindungsgemäßen Verbindung entsprechen 10 ml orale Suspension.

**Herstellung:** Das Rhodigel wird in Ethanol suspendiert, der Wirkstoff wird der Suspension zugefügt. Unter Rühren erfolgt die Zugabe des Wassers. Bis zum Abschluss der Quellung des Rhodigels wird ca. 6 h gerührt.

## Patentansprüche

1. Verbindung der Formel 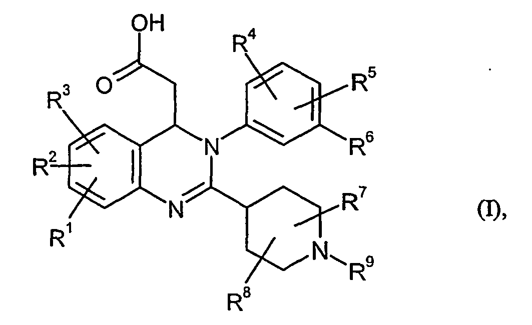 in welcher
R¹, R² und R³ unabhängig voneinander für Wasserstoff, Allyl, Alkoxy, Carboxyl, Alkyl-carbonyl, Alkoxycarbonyl, Aminocarbonyl, Trifluormethyl, Halogen, Cyano, Hydroxy oder Nitro stehen,
R⁴ und R⁵ unabhängig voneinander für Wasserstoff, Alkyl, Alkoxy, Alkylthio, Cyano, Halogen, Nitro, Trifluormethyl oder Trifluormethoxy stehen,
R⁶ für Alkyl, Cyano, Halogen, Nitro oder Trifluormethyl steht,
R⁷ und R⁸ unabhängig voneinander für Wasserstoff, Halogen, Alkyl oder Alkoxy, stehen und
R⁹ für Aryl oder 1,3-Benzodioxol-5-yl steht, worin Aryl und 1,3-Benzodioxol-5-yl substituiert sein können mit 1 bis 3 Substituenten, wobei die Substituenten unabhängig voneinander ausgewählt werden aus der Gruppe, bestehend aus Alkoxy, Alkylthio, Carboxyl, Alkylcarbonyl, Alkoxycarbonyl, Aminocarbonyl, Trifluormethyl, Halogen, Carbamoyl, Cyano, Hydroxy, Amino, Alkylamino, Nitro und gegebenenfalls Hydroxy-substituiertes Alkyl,
oder eines ihrer Salze, ihrer Solvate oder der Solvate ihrer Salze.

2. Verbindung nach Anspruch 1, **dadurch gekennzeichnet, dass**
R¹, R² und R³ unabhängig voneinander für Wasserstoff, Methyl, Fluor, Chlor, Cyano, Hydroxy oder Aminocarbonyl stehen,
R⁴ und R⁵ unabhängig voneinander für Wasserstoff, Fluor, C₁-C₄-Alkyl oder C₁-C₄-Alkoxy stehen,
R⁶ für Chlor, Nitro, Trifluormethyl, Methyl, iso-Propyl oder tert.-Butyl steht,
R⁷ und R⁸ unabhängig voneinander für Wasserstoff, oder C₁-C₃-Alkyl stehen und
R⁹ für Phenyl oder 1,3-Benzodioxol-5-yl steht, worin Phenyl substituiert sein kann mit 1 bis 3 Substituenten, wobei die Substituenten unabhängig voneinander ausgewählt werden aus der Gruppe, bestehend aus C₁-C₆-Alkyl, C₁-C₆-Alkoxy, Carboxyl, C₁-C₆-Alkylcarbonyl, C₁-C₆-Alkoxycarbonyl, Trifluormethyl, Fluor, Chlor, Brom, Cyano, Hydroxy, Amino, C₁-C₆-Alkylamino und Nitro.

3. Verbindung nach Anspruch 1 oder 2, **dadurch gekennzeichnet, dass**
R¹ und R² für Wasserstoff stehen,
R³ für Fluor steht,
R⁴ und R⁵ unabhängig voneinander für Wasserstoff, Fluor oder Methoxy stehen,
R⁶ für Trifluormethyl steht,
R⁷ und R⁸ für Wasserstoff stehen und
R⁹ für Phenyl steht, worin Phenyl substituiert sein kann mit 1 oder 2 Substituenten, wobei die Substituenten unabhängig voneinander ausgewählt werden aus der Gruppe, bestehend aus Methyl, Methoxy, Ethoxy, Fluor und Chlor.

4. Verfahren zur Herstellung einer Verbindung der Formel (I) nach Anspruch 1, **dadurch gekennzeichnet, dass** eine Verbindung der Formel 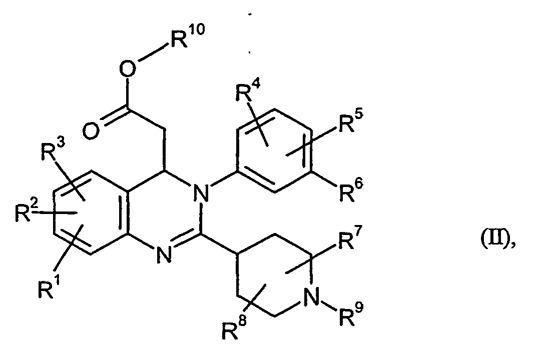 in welcher
R¹, R², R³, R⁴, R⁵, R⁶, R⁷, R⁸ und R⁹ die in Anspruch 1 angegebene Bedeutung haben, und
R¹⁰ für Alkyl, bevorzugt Methyl oder Ethyl, steht,
mit einer Base umgesetzt wird.

5. Verbindung nach einem der Ansprüche 1 bis 3 zur Behandlung und/oder Prophylaxe von Krankheiten.

6. Verwendung einer Verbindung nach einem der Ansprüche 1 bis 3 zur Herstellung eines Arzneimittels zur Behandlung und/oder Prophylaxe von Krankheiten.

7. Verwendung einer Verbindung nach einem der Ansprüche 1 bis 3 zur Herstellung eines Arzneimittels zur Behandlung und/oder Prophylaxe von Virusinfeh-tionen.

8. Verwendung nach Anspruch 7, **dadurch gekennzeichnet, dass** die Virusinfektion eine Infektion mit dem humanen Cytomegalovirus (HCMV) oder einem anderen Vertreter der Gruppe der Herpes viridae ist.

9. Arzneimittel enthaltend eine Verbindung, wie in einem der Ansprüche 1 bis 3 definiert, in Kombination mit einem weiteren Wirkstoff.

10. Arzneimittel enthaltend eine Verbindung nach einem der Ansprüche 1 bis 3 in Kombination mit einem inerten, nichttoxischen, pharmazeutisch geeigneten Hilfsstoff.

11. Arzneimittel nach Anspruch 10 zur Behandlung und/oder Prophylaxe von Virusinfektionen.

## Claims

1. Compound of the formula 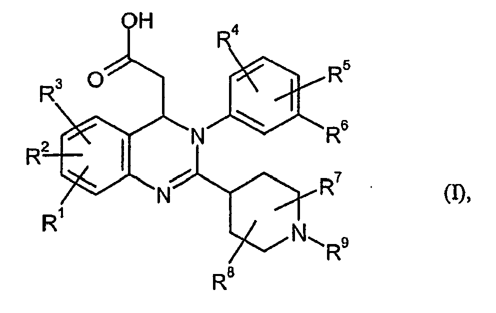 in which
R¹, R² and R³ independently of one another represent hydrogen, alkyl, alkoxy, carboxyl, alkylcarbonyl, alkoxycarbonyl, aminocarbonyl, trifluoromethyl, halogen, cyano, hydroxyl or nitro,
R⁴ and R⁵ independently of one another represent hydrogen, alkyl, alkoxy, alkylthio, cyano, halogen, nitro, trifluoromethyl or trifluoromethoxy,
R⁶ represents alkyl, cyano, halogen, nitro or trifluoromethyl,
R⁷ and R⁸ independently of one another represent hydrogen, halogen, alkyl or alkoxy and
R⁹ represents aryl or 1,3-benzodioxol-5-yl, where aryl and 1,3-benzodioxol-5-yl may be substituted by 1 to 3 substituents, where the substituents independently of one another are selected from the group consisting of alkoxy, alkylthio, carboxyl, alkylcarbonyl, alkoxycarbonyl, aminocarbonyl, trifluoromethyl, halogen, carbamoyl, cyano, hydroxyl, amino, alkylamino, nitro and optionally hydroxyl-substituted alkyl,
or one of its salts, its solvates or the solvates of its salts.

2. Compound according to Claim 1, **characterized in that**
R¹, R² and R³ independently of one another represent hydrogen, methyl, fluorine, chlorine, cyano, hydroxyl or aminocarbonyl,
R⁴ and R⁵ independently of one another represent hydrogen, fluorine, C₁-C₄-alkyl or C₁-C₄-alkoxy,
R⁶ represents chlorine, nitro, trifluoromethyl, methyl, isopropyl or tert-butyl,
R⁷ and R⁸ independently of one another represent hydrogen or C₁-C₃-alkyl and
R⁹ represents phenyl or 1,3-benzodioxol-5-yl, where phenyl may be substituted by 1 to 3 substituents, where the substituents independently of one another are selected from the group consisting of C₁-C₆-alkyl, C₁-C₆-alkoxy, carboxyl, C₁-C₆-alkylcarbonyl, C₁-C₆-alkoxycarbonyl, trifluoromethyl, fluorine, chlorine, bromine, cyano, hydroxyl, amino, C₁-C₆-alkylamino and nitro.

3. Compound according to Claim 1 or 2, **characterized in that**
R¹ and R² are hydrogen,
R³ is fluorine,
R⁴ and R⁵ independently of one another are hydrogen, fluorine or methoxy,
R⁶ is trifluoromethyl,
R⁷ and R⁸ are hydrogen and
R⁹ is phenyl, where phenyl may be substituted by 1 or 2 substituents, where the substituents independently of one another are selected from the group consisting of methyl, methoxy, ethoxy, fluorine and chlorine.

4. Process for preparing a compound of the formula (I) according to Claim 1, **characterized in that** a compound of the formula 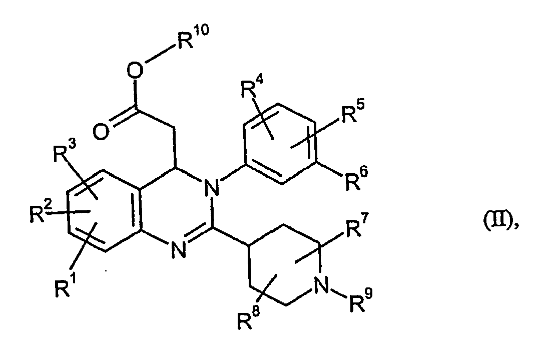 in which
R¹, R², R³ , R⁴, R⁵, R⁶, R⁷, R⁸ and R⁹ are as defined in Claim 1, and
R¹⁰ represents alkyl, preferably methyl or ethyl,
is reacted with a base.

5. Compound according to any of Claims 1 to 3 for the treatment and/or prophylaxis of diseases.

6. Use of a compound according to any of Claims 1 to 3 for preparing a medicament for the treatment and/or prophylaxis of diseases.

7. Use of a compound according to any of Claims 1 to 3 for preparing a medicament for the treatment and/or prophylaxis of viral infections.

8. Use according to Claim 7, **characterized in that** the viral infection is an infection with the human cytomegalovirus (HCMV) or another representative of the group of the Herpes viridae.

9. Medicament, comprising a compound as defined in any of Claims 1 to 3 in combination with a further active compound.

10. Medicament, comprising a compound according to any of Claims 1 to 3 in combination with an inert nontoxic, pharmaceutically acceptable auxiliary.

11. Medicament according to Claim 10 for the treatment and/or prophylaxis of viral infections.

## Revendications

1. Composé de formule 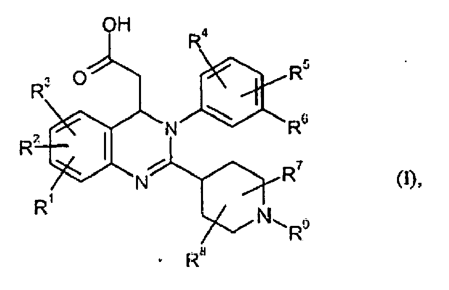 dans laquelle
R¹, R² et R³ représentent, indépendamment les uns des autres, l'hydrogène, un reste alkyle, alkoxy, carboxyle, alkylcarbonyle, alkoxycarbonyle, aminocarbonyle, trifluorométhyle, halogéno, cyano, hydroxy ou nitro,
R⁴ et R⁵ représentent, indépendamment l'un de l'autre, l'hydrogène, un reste alkyle, alkoxy, alkylthio, cyano, halogéno, nitro, trifluorométhyle ou trifluorométhoxy,
R⁶ représente un reste alkyle, cyano, halogéno, nitro ou trifluorométhyle,
R⁷ et R⁸ représentent, indépendamment l'un de l'autre, l'hydrogène, un reste halogéno, alkyle ou alkoxy et
R⁹ représente un reste aryle ou 1,3-benzodioxole-5-yle, les restes aryle et 1,3-benzodioxole-5-yle pouvant être substitués avec 1 à 3 substituants choisis indépendamment les uns des autres dans le groupe constitué des restes alkoxy, alkylthio, carboxyle, alkylcarbonyle, alkoxycarbonyle, aminocarbonyle, trifluorométhyle, halogéno, carbamoyle, cyano, hydroxy, amino, alkylamino, nitro et alkyle éventuellement substitué par un radical hydroxy,
ou l'un de ses sels, de ses produits de solvatation ou des produits de solvatation de ses sels.

2. Composé suivant la revendication 1, **caractérisé en ce que**
R¹, R² et R³ représentent, indépendamment les uns des autres, l'hydrogène, un reste méthyle, fluoro, chloro, cyano, hydroxy ou aminocarbonyle,
R⁴ et R⁵ représentent, indépendamment l'un de l'autre, l'hydrogène, un reste fluoro, alkyle en C₁ à C₄ ou alkoxy en C₁ à C₄,
R⁶ représente un reste chloro, nitro, trifluorométhyle, méthyle, isopropyle ou tertio-butyle,
R⁷ et R⁸ représentent, indépendamment l'un de l'autre, l'hydrogène ou un reste alkyle en C₁ à C₃,
R⁹ représente un reste phényle ou 1,3-benzodioxole-5-yle, le reste phényle pouvant être substitué avec 1 à 3 substituants choisis indépendamment les uns des autres dans le groupe constitué de restes alkyle en C₁ à C₆, alkoxy en C₁ à C₆, carboxyle, (alkyle en C₁ à C₆)-carbonyle, (alkoxy en C₁ à C₆) carbonyle, trifluorométhyle, fluoro, chloro, bromo, cyano, hydroxy, amino, alkylamino en C₁ à C₆ et nitro.

3. Composé suivant la revendication 1 ou 2,
**caractérisé en ce que**
R¹ et R² représentent l'hydrogène,
R³ représente un reste fluoro,
R⁴ et R⁵ représentent, indépendamment l'un de l'autre, l'hydrogène, un reste fluoro ou méthoxy,
R⁶ est un reste trifluorométhyle,
R⁷ et R⁸ représentent l'hydrogène et
R⁹ représente un reste phényle, le reste phényle pouvant être substitué avec 1 ou 2 substituants choisis indépendamment l'un de l'autre dans le groupe constitué des restes méthyle, méthoxy, éthoxy, fluoro et chloro.

4. Procédé de production d'un composé de formule (I) suivant la revendication 1, **caractérisé en ce qu'**un composé de formule 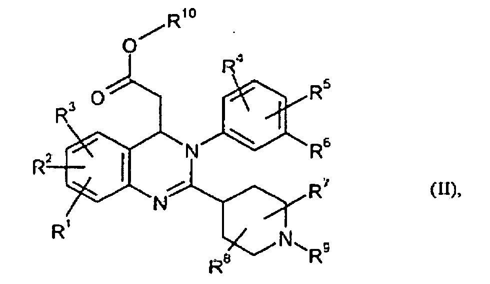 dans laquelle
R¹, R², R³, R⁴, R⁵, R⁶, R⁷, R⁸ et R⁹ ont la définition indiquée dans la revendication 1 et
R¹⁰ représente un reste alkyle, avantageusement méthyle ou éthyle,
est amené à réagir avec une base.

5. Composé suivant l'une des revendications 1 à 3 destiné au traitement et/ou à la prophylaxie de maladies.

6. Utilisation d'un composé suivant l'une des revendications 1 à 3 pour la préparation d'un médicament destiné au traitement et/ou à la prophylaxie de maladies.

7. Utilisation d'un composé suivant l'une des revendications 1 à 3 pour la préparation d'un médicament destiné au traitement et/ou à la prophylaxie d'infections virales.

8. Utilisation suivant la revendication 7, **caractérisée en ce que** l'infection virale est une infection causée par le cytomégalovirus humain (HCMV) ou par un autre représentant du groupe des Herpes viridae.

9. Médicament contenant un composé tel que défini dans l'une des revendications 1 à 3 en combinaison avec une autre substance active.

10. Médicament contenant un composé suivant l'une des revendications 1 à 3 en combinaison avec une substance auxiliaire inerte non toxique pharmaceutiquement acceptable.

11. Médicament suivant la revendication 10, destiné au traitement et/ou à la prophylaxie d'infections virales.
